# EUROPEAN PATENT APPLICATION

(11) **EP 3 696 159 A1**
(43) Date of publication of application: **19.08.2020**
(21) Application number: 19157232.0
(22) Date of filing: 14.02.2019
(51) Int. Cl.: C07C 45/00, C07C 49/84, C07D 249/08

(54) **PROCESS FOR THE PREPARATION OF SUBSTITUTED PHENOXYPHENYL KETONES**

(71) Applicant: BASF Agro B.V., 6835 EA Arnhem (NL)
(72) Inventor: The designation of the inventor has not yet been filed
(74) Representative: BASF IP Association

(57) **Abstract**

The present invention relates to a process for providing substituted phenoxyphenyl ketones and to a process for conventing them to substituted 1-[4-phenoxy-2-(halogenalkyl)phenyl]-2-(1,2,4-triazol-1-yl)ethanol compounds.

## Description

The present invention relates to a process for providing substituted phenoxyphenyl ketones and to a process for converting them to substituted 1-[4-phenoxy-2-(halogenalkyl)phenyl]-2-(1,2,4-triazol-1-yl)ethanol compounds.

WO 2013/007767 describes the above mentioned triazole compounds, their fungicidal activity and their synthesis via substituted phenoxyphenyl ketones. These compounds are highly efficient fungicides. Therefore, there is an ongoing need for processes that easily make them available.

WO 2014/108286, WO 2015/091045, WO2016/005211 and WO 2016/202807 describe process steps and further processes for the synthesis of substituted 1-[4-phenoxy-2-(halogenalkyl)-phenyl]-2-(1,2,4-triazol-1-yl)ethanol compounds.

The present invention provides a process for the preparation of substituted phenoxyphenyl ketones and fungicidal 1-[4-phenoxy-2-(halogenalkyl)phenyl]-2-(1,2,4-triazol-1-yl)ethanol derivatives utilizing a different starting material. Hence, the present invention provides for a process for the preparation of substituted phenoxyphenyl ketones and the fungicidal triazole compounds via a different efficient synthetic route.

It was an object of the present invention to provide an industrially simple process for the preparation of substituted phenoxyphenyl ketones in good yields. In addition, the process should be environmentally friendly in order to reduce unfavorable environmental effects. A further object of the present invention was to provide an improved process for the synthesis of 1-[4-phenoxy-2-(halogenalkyl)phenyl]-2-(1,2,4-triazol-1-yl)ethanol compounds.

It has now surprisingly been found a highly efficient process for the synthesis of substituted phenoxyphenyl ketones, and thus, an efficient synthesis of triazole compounds with fungicidal activity.

The present invention thus relates to a process for the preparation of the compounds (V) 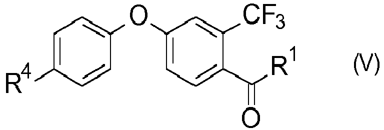 comprising the following step:
(iii) reacting a nitrile compound of formula (IV) 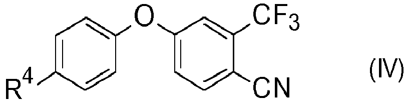 with compounds selected from the group comprising R¹-Mg-Hal, (R¹)Li, (R¹)₂Zn and Al(R¹)₃,
wherein the variables are defined as follows:
- R¹: is C₁-C₆-alkyl or C₃-C₈-cycloalkyl;
- R⁴: is halogen; and
- Hal: is halogen.

The compounds of formula (V) are valuable intermediates for the synthesis of triazole compounds having fungicidal activity.

The present invention thus further relates to a process for the preparation of triazole compounds of the formula (T): 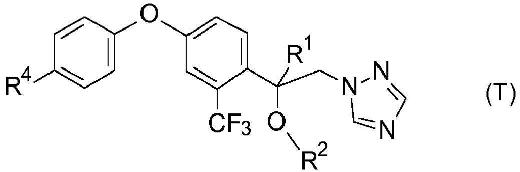
wherein R¹ and R⁴ are as described for the compounds of formula (V),
R² is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl; wherein the aliphatic moieties of R² are not further substituted or do carry one, two, three or up to the maximum possible number of identical or different groups R^{2a} which independently are selected from:
   R^{2a} halogen, OH, CN, nitro, C₁-C₄-alkoxy, C₃-C₈-cycloalkyl, C₃-C₈-halocycloalkyl and C₁-C₄-halogenalkoxy;
   wherein the cycloalkyl and/or phenyl moieties of R² are not further substituted or do carry one, two, three, four, five or up to the maximum number of identical or different groups R^{2b} which independently are selected from:
      R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
comprising the following steps:
(iii) as described herein to obtain the compound of formula (V);
(iv) converting the ketone of the formula (V) to oxiranes (O); 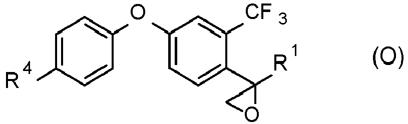
(v) reacting the oxirane (O) with 1H-1,2,4-triazole in the presence of a base to obtain compounds (T), wherein R² is hydrogen (compounds T-1);
and, for obtaining compounds wherein R² is different from hydrogen:
a) derivatizing the compound of formula (T-1) of step (iii) under basic conditions with R²-LG, wherein LG is a nucleophilically replaceable leaving group; to result in compounds (T-2).

Further embodiments of the invention are evident from the claims, the description and the examples. It is to be understood that the single features of the subject matter of the invention described herein can be applied not only in the combination given in each particular case but also in other combinations, without leaving the scope of the invention.

In the definitions of the variables given herein, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert-butyl).

The term "C₂-C₆-alkenyl" refers to a straight-chain or branched unsaturated hydrocarbon radical having 2 to 6 carbon atoms and a double bond in any position, e.g. ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl. Likewise, the term "C₂-C₆-alkenyl" refers to a straight-chain or branched unsaturated hydrocarbon radical having 2 to 6 carbon atoms and a double bond in any position.

The term "C₂-C₆-alkynyl" refers to a straight-chain or branched unsaturated hydrocarbon radical having 2 to 6 carbon atoms and containing at least one triple bond, such as ethynyl, prop-1-ynyl, prop-2-ynyl (propargyl), but-1-ynyl, but-2-ynyl, but-3-ynyl, 1-methyl-prop-2-ynyl. Likewise, the term "C₂-C₆-alkynyl" refers to a straight-chain or branched unsaturated hydrocarbon radical having 2 to 6 carbon atoms and at least one triple bond.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-halocycloalkyl" or "C₃-C₈-halogencycloalkyl" refers to monocyclic saturated hydrocarbon radicals having 3 to 8 carbon ring members as defined above, wherein some or all of the hydrogen atoms in these groups may be replaced by halogen atoms as mentioned above.

The term "C₃-C₆-cycloalkyl-C₁-C₆-alkyl" refers to alkyl having 1 to 6 carbon atoms (as defined above), wherein one hydrogen atom of the alkyl radical is replaced by a cycloalkyl radical having 3 to 6 carbon atoms (as defined above).

The term "C₁-C₆-alkoxy" refers to a straight-chain or branched alkyl group having 1 to 6 carbon atoms which is bonded via an oxygen at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-haloalkoxy" or "C₁-C₆-halogenalkoxy" refers to a C₁-C₆-alkoxy radical as defined above, wherein some or all of the hydrogen atoms in these groups may be replaced by halogen atoms as mentioned above. A preferred embodiment of a C₁-C₆-haloalkoxy is a C₁-C₄-haloalkoxy. Examples of C₁-C₄-haloalkoxy groups include substituents, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, OCF₂CHF₂, OCHF-CF₃, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F5, OCF₂-C₂F5, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "phenyl-C₁-C₄-alkyl" refers to alkyl having 1 to 6 carbon atoms (as defined above), wherein one hydrogen atom of the alkyl radical is replaced by a phenyl radical. Likewise, the terms "phenyl-C₂-C₄-alkenyl" and "phenyl-C₂-C₄-alkynyl" refer to alkenyl and alkynyl, respectively, wherein one hydrogen atom of the aforementioned radicals is replaced by a phenyl radical.

According to step (iii) of the process, compounds (IV) are reacted with a compound selected from the group comprising R¹-Mg-Hal (G1), (R¹)Li(G2), (R¹)₂Zn (G3) and Al(R¹)₃ (G4), wherein
- R¹: is C₁-C₆-alkyl or C₃-C₈-cycloalkyl;
- Hal: is halogen.

R¹ in said compounds is C₁-C₆-alkyl or C₃-C₆-cycloalkyl. Preferably it is selected from methyl, ethyl, n-propyl, i-propyl, n-butyl, t-butyl, sec-butyl and cyclopropyl; more preferably from methyl, i-propyl, t-butyl, sec-butyl and cyclopropyl.

According to one embodiment, R¹ is C₁-C₆-alkyl, more specifically C₁-C₄-alkyl, preferably it is selected from CH₃, C₂H₅, n-C₃H₇, CH(CH₃)₂, n-butyl, iso-butyl and tert-butyl; more preferably from CH₃, C₂H₅, CH(CH₃)₂ and C(CH₃)₃; most preferably from CH₃ or CH(CH₃)₂. In one particularly preferred embodiment, R¹ is CH₃. In another particularly preferred embodiment, R¹ is CH(CH₃)₂.

According to a further embodiment, R¹ is C₃-C₆-cycloalkyl, such as C₃H₅ (cyclopropyl), C₄H₇ (cyclobutyl), cyclopentyl or cyclohexyl. Preferably, R¹ is C₃H₅ (cyclopropyl) or C₄H₇ (cyclobutyl), more preferably cyclopropyl.

Hal stands for halogen, preferably for Cl or Br.

According to one embodiment, compounds (IV) are reacted with a Grignard reagent R¹-Mg-Hal (G1).

More than one Grignard reagent can be used in the same reaction. For example, two different reagents (G1), wherein R¹ of both reagents is the same, Hal of the one reagent is Br and Hal of the second reagents is Cl, can be used.

Preferably, (G1) is used in an amount of 1 eq to 3 eq, in particular 1.1 to 2.5 eq, more specifically 1.2 to 2.2 eq, in relation to one equivalent of compound (IV). In particular, the amounts of 1.4 to 2.2 eq, more particularly 1.2 to 2.2 eq per mole of compound (IV) may be favorable according to the present invention. Usually, the Grignard reagent is used in excess.

As generally known to the skilled person, the structure of a Grignard reagent can be described by the so-called Schlenck equilibrium. A Grignard reagent undergoes a solvent-dependent equilibrium between different magnesium compounds. The Schlenck equilibrium for the Grignard reagent used according to the present invention can be schematically illustrated as follows:

2 R¹-Mg-Hal ⇄ (R¹)₂Mg + Mg(Hal)₂ ⇄ (R¹)₂Mg ● Mg(Hal)₂

As known, depending on the solvent used in the inventive reaction, solvent molecules may add to the Mg-reagents, thereby forming - in case of the use of ethers - the respective etherates.

For general information regarding structures of Grignard reagents, see also Milton Orchin, Journal of Chemical Education, Volume 66, Number 7, 1999, pp 586 to 588.

According to one embodiment, LiCI is added to the reaction mixture of step (iii). According to a specific embodiment, before contacting the Grignard reagent (G1) with the reagents of the inventive process, it is brought together with LiCI, thereby forming an addition product R¹MgHal ● LiCl ((G1)●LiCl). According to this embodiment, ((G1)●LiCl) is then used in step (iii). The use of LiCI together with Grignard reagents is generally known in the art, see for example Angew. Chem. Int. Ed. 2004, 43, 3333 and Angew. Chem. Int. Ed. 2006, 45, 159.

The Grignard reagents (G1) or their addition products with LiCl ((G1)●LiCl) are commercially available or can be made according to processes well-known to the skilled person (see Angew. Chem. Int. Ed. 2004, 43, 3333).

The Grignard reagent is added in the manner as is common to the skilled person. In particular, it can be added as solution in an appropriate solvent such as tetrahydrofurane (THF), 1,4-dioxane, diethylether, methyl-t-butylether (MTBE) and 2-methyl-tetrahydrofurane.

According to another embodiment, compounds (IV) are reacted with a reagent of the (R¹)Li (G2).

Preferably, (G2) is used in an amount of 1 eq to 3 eq, in particular 1.1 to 2.5 eq, more specifically 1.2 to 2.2 .2eq, in relation to one equivalent of compound (IV). In particular, the amounts of 1.4 to 2.2 eq, more particularly 1.2 to 2.2 eq per mole of compound (IV) may be favorable according to the present invention. Usually, the reagent is used in excess.

As known, depending on the solvent used in the inventive reaction, solvent molecules may add to the Li-reagents, thereby forming - in case of the use of ethers - the respective etherates.

The reagents (G2) are commercially available or can be made according to processes well-known to the skilled person.

The reagent (G2) is added in the manner as is common to the skilled person. In particular, it can be added as solution in an appropriate solvent such as tetrahydrofurane (THF), 1,4-dioxane, diethylether, methyl-t-butylether (MTBE) and 2-methyl-tetrahydrofurane.

According to another embodiment, compounds (IV) are reacted with a reagent of the (R¹)₂Zn (G3).

Preferably, (G3) is used in an amount of 0.5 eq to 3 eq, in particular 0.6 to 2.5 eq, more specifically 0.65 to 2.2 eq, in relation to one equivalent of compound (IV). In particular, the amounts of 0.7 to 2.2 eq, more particularly 0.7 to 2.0 eq per mole of compound (IV) may be favorable according to the present invention. Usually, the reagent is used in excess.

As known, depending on the solvent used in the inventive reaction, solvent molecules may add to the Zn-reagents, thereby forming - in case of the use of ethers - the respective etherates.

The reagents (G3) are commercially available or can be made according to processes well-known to the skilled person.

The reagent (G3) is added in the manner as is common to the skilled person. In particular, it can be added as solution in an appropriate solvent such as tetrahydrofurane (THF), 1,4-dioxane, diethylether, methyl-t-butylether (MTBE) and 2-methyl-tetrahydrofurane.

According to another embodiment, compounds (IV) are reacted with a reagent of the Al(R¹)₃ (G4).

Preferably, (G4) is used in an amount of 0.33 eq to 3 eq, in particular 0.4 to 2.5 eq, more specifically 0.5 to 2.2 eq, in relation to one equivalent of compound (IV). In particular, the amounts of 0.5 to 2.0 eq, more particularly 0.5 to 1.5 eq per mole of compound (IV) may be favorable according to the present invention. Usually, the reagent is used in excess.

As known, depending on the solvent used in the inventive reaction, solvent molecules may add to the Al-reagents, thereby forming - in case of the use of ethers - the respective etherates.

The reagents (G4) are commercially available or can be made according to processes well-known to the skilled person.

The reagent (G4) is added in the manner as is common to the skilled person. In particular, it can be added as solution in an appropriate solvent such as tetrahydrofurane (THF), 1,4-dioxane, diethylether, methyl-t-butylether (MTBE) and 2-methyl-tetrahydrofurane.

The process step (iii) according to the present invention can be carried out in the presence of a catalyst. The catalyst is preferably selected from Cu or Ti catalysts.

According to one embodiment, the catalyst is a Cu catalyst, preferably CuBr, CuBr₂, CuCl, CuCl₂, CuI, Cu(OAc)₂ or CuCN.

According to another embodiment, the catalyst is a Ti catalyst, preferably Ti(OiPr)₄.

The molar ratio of the catalyst to compound (IV) is generally in the range from 0.001:1 to 0.5:1, preferably from 0.005:1 to 0.2:1, more preferably from 0.01:1 to 0.1:1, most preferably from 0.01:1 to 0.05:1.

Examples for appropriate solvents for step (iii) of the inventive process are aprotic organic solvents such as for example diethylether, tetrahydrofurane (THF), methyl-tert-butylether (MTBE), toluene, ortho-xylene, meta-xylene, para-xylene and mixtures thereof. Typically, the Grignard reagent is added as solution in THF, 1,4-dioxane, diethylether or 2-methyl-tetrahydrofurane (2-Me-THF), in particular in THF or diethylether, to the reaction vessel or flask containing the reagent (IV) and a solvent such as, for example, toluene, MTBE, ortho-xylene, meta-xylene, para-xylene, mesitylene and/or diisopropylether, in particular toluene, MTBE and/or ortho-xylene.

The temperature for the reaction of the Grignard reagent in step (iii) may be from -20°C to 70°C, preferably from 0°C to 70°C, more preferably from 25 °C to 70 °C, most preferably at 35 to 65 °C and is preferably held at a maximum of 65°C, in particular at a maximum of 60°C.

After step (iii), a work-up of the reaction mixture can be carried out by procedures known in a general manner to the person skilled in the art. For example, after completion of the reaction, acid or aqueous acidic solution is added. Sometimes it is practicable to add the reaction mixture to acid or aqueous acidic solution after completion of the reaction followed by the respective further work up. Thereafter, the organic phases are washed with water and the solvent is removed from the separated organic phases.

Further, it may be appropriate to wash the organic phases with acidic or basic aqueous solution instead or in addition to washing with water.

The so-obtained raw product can be directly used in the next process step, i.e. step (iv) of the inventive process. However, the raw product can also be further worked up and/or purified as generally known to the skilled person. If this is deemed appropriate, the reaction mixture is extracted with a suitable organic solvent (for example aromatic hydrocarbons such as toluene and xylenes) and the residue is, if appropriate, purified by recrystallization and/or chromatography.

The compounds of formula (IV) can be prepared, e.g., by a process comprising the following steps:
(i) reacting a compound of formula (II) 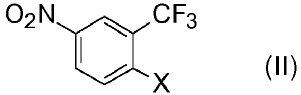
   wherein X is halogen;
   with a cyanide in the form of an inorganic salt, a cyano-metal complex salt or a hydrate thereof, or an organic cyanohydrin;
   to give the compound of formula (I) 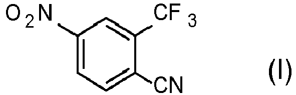
(ii) reacting the compound of formula (I) with a phenol derivative of formula (III) 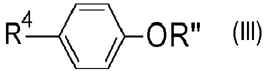 wherein
   - R": is hydrogen or an alkali or earth alkali metal ion;
   - R⁴: is as defined for compounds of formula (IV);
   in the presence of a base if R" is hydrogen.

The compounds of formula (II) can be synthesized as known to the skilled person or are also commercially available.

The variable X of the compound of formula (II) used in step (i) is halogen. X is preferably selected from Br or Cl, more preferably X is Cl. According to one specific embodiment, X is Br. According to another specific embodiment, X is Cl.

In the process step (i), the compound (II) is reacted with a cyanide. The cyanide is used in the form of an inorganic salt, a cyano-metal complex salt including hydrates thereof or an organic cyanohydrin.

According to one embodiment, in step (i) the cyanide is used in the form of an inorganic salt. The inorganic salt is preferably selected from KCN, NaCN, CuCN, Ca(CN)₂, Zn(CN)₂, Co(CN)₂ or any mixtures thereof; more preferably from NaCN, KCN, Zn(CN)₂ or any mixtures thereof; most preferably the inorganic salt is Zn(CN)₂.

According to one specific embodiment, the inorganic salt is KCN.

According to another specific embodiment, the inorganic salt is NaCN.

According to another specific embodiment, the inorganic salt is CuCN.

According to another specific embodiment, the inorganic salt is Ca(CN)₂.

According to another specific embodiment, the inorganic salt is Zn(CN)₂.

According to another specific embodiment, the inorganic salt is Co(CN)₂

According to another embodiment, in step (i) the cyanide is used in the form of a cyano-metal complex salt including hydrates thereof. The cyano-metal complex salt preferably has the general formula (CM)

M¹ₐ[M²(CN)ₓ] (CM)

wherein
- M¹: is selected from K, Na, Cu(I), Cu(II), Ca, Zn;
- M²: is selected from Fe(II), Fe (III), Co(II), Co(III), Mn(II), Mn(III), Ni, Cr(II), Cr(III);
- a: is 2, 3 or 4;
- x: is 2, 4 or 6.

The variables a and x are selected in a way that the cyano-metal complex salt is neutrally charged.

The cyano-metal complex salts of formula (CM) can be synthesized as known to the skilled person or are also at least partially commercially available.

M¹ in cyano-metal complex salts of formula (CM) is selected from K, Na, Cu(I), Cu(II), Ca, Zn; preferably from K or Na, more preferably M¹ is K.

M² in cyano-metal complex salts of formula (CM) is selected from Fe(II), Fe (III), Co(II), Co(III), Mn(II), Mn(III), Ni, Cr(II), Cr(III); preferably from Fe(II) or Fe (III), more preferably M² is Fe(II).

According to one specific embodiment, M¹ is selected from K or Na and M² is selected from Fe(II) or Fe (III).

According to a further specific embodiment, M¹ is K and M² is selected from Fe(II) or Fe (III).

According to a still further specific embodiment, M¹ is Na and M² is selected from Fe(II) or Fe (III).

According to a still further specific embodiment, M¹ is selected from K or Na and M² is Fe(II).

According to a still further specific embodiment, M¹ is selected from K or Na and M² is Fe(III).

The cyano-metal complexes are preferably selected from K₄[Fe(CN)₆], K₃[Fe(CN)₆], Na₄[Fe(CN)₆], Na₃[Fe(CN)₆] or any hydrates thereof; more preferably from K₄[Fe(CN)₆], K₃[Fe(CN)₆] or any hydrates thereof; most preferably it is K₄[Fe(CN)₆] or any hydrates thereof.

According to one specific embodiment, the cyano-metal complex is K₄[Fe(CN)₆] or any hydrates thereof.

According to another specific embodiment, the cyano-metal complex is K₃[Fe(CN)₆] or any hydrates thereof.

According to another specific embodiment, the cyano-metal complex is Na₄[Fe(CN)₆] or any hydrates thereof.

According to another specific embodiment, the cyano-metal complex is Na₃[Fe(CN)₆] or any hydrates thereof.

According to still another embodiment, in step (i) the cyanide is used in the form of organic cyanohydrines. The cyanohydrins preferably have the general formula (CH) 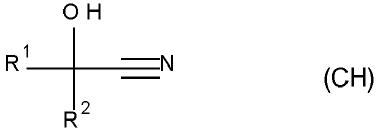 wherein R¹ and R² are independently selected from (C₁-C₆)-alkyl, (C₂-C₆)-alkenyl, (C₃-C₆)-cyclyalkyl or phenyl.

The cyanohydrines of formula (CH) can be synthesized as known to the skilled person or are also commercially available.

The cyanide (an inorganic salt, a cyano-metal complex salt including hydrates thereof or an organic cyanohydrin) is usually used in an amount from 0.2 to 4 mole equivalents of a cyanide compound per 1 mole of compound (II), preferably from 0.2 to 3 mole equivalents of a cyanide compound per 1 mole of compound (II), more preferably from 0.2 to 2 mole equivalents of a cyanide compound per 1 mole of compound (II), most preferably from 0.2 to 1.5 mole equivalents of a cyanide compound per 1 mole of compound (II).

The cyanide (an inorganic salt, a cyano-metal complex salt including hydrates thereof or an organic cyanohydrin) can be also used in an amount from 0.2 to 1 mole equivalents of a cyanide compound per 1 mole of compound (II), preferably from 0.3 to 0.9 mole equivalents of a cyanide compound per 1 mole of compound (II), more preferably from 0.4 to 0.8 mole equivalents of a cyanide compound per 1 mole of compound (II), most preferably from 0.4 to 0.7 mole equivalents of a cyanide compound per 1 mole of compound (II).

The cyanide (an inorganic salt, a cyano-metal complex salt including hydrates thereof or an organic cyanohydrin) is usually used in an amount from 0.8 to 3.0 CN-equivalents per 1 mole of compound (II), preferably from 0.8 to 2.0 CN-equivalents per 1 mole of compound (II), more preferably from 1.0 to 2.0 CN-equivalents per 1 mole of compound (II), most preferably from 1.0 to 1.4 CN-equivalents per 1 mole of compound (II).

The cyanide can be added to the reaction mixture in one portion or gradually. According to one embodiment it is added to the reaction mixture in one portion. According to another embodiment it is added to the reaction mixture gradually.

The process step (i) can be carried out in the presence of a catalyst or a precatalyst.

According to one embodiment, the reaction of step (i) is carried out in the presence of a catalyst. The catalyst is preferably selected from Pd, Cu(I), Cu(II) and Ni catalysts.

These catalysts can be synthesized as known to the skilled person or are also commercially available.

According to one specific embodiment, the catalyst is a Pd catalyst. Preferably, the Pd catalyst is a Pd complex with phosphorous containing ligands.

Usually said Pd complexes are prepared *in situ* by mixing a Pd(0) or Pd(II) source with a desired ligand.

The molar ratio of Pd source to the ligand is usually in the range from 1:1 to 1:4, preferably from 1:1 to 1:3, more preferably from 1:1 to 1: 2. According to one specific embodiment, the ratio is 1:1. According to another specific embodiment the ratio is 1:2.

The Pd source used in the process step (i) according to the present invention is selected from tris(dibenzylideneacetone)dipalladium (0) (Pd₂(dba)₃); bis[cinnamyl palladium (II) chloride] ([(Cinnamyl)PdCl]₂); palladium (II) acetate (Pd(OAc)₂); or palladium (II) chloride (PdCl₂).

According to one embodiment, Pd source is Pd₂(dba)₃.

According to another embodiment, Pd source is [(Cinnamyl)PdCl]₂.

According to another embodiment, Pd source is Pd(OAc)₂.

According to another embodiment, Pd source is PdCl₂.

The ligands of the palladium complex with phosphorous containing ligands used in the process step (i) according to the present invention have the general formula (L)

PR^{P1}R^{P2}R^{P3} (L),

wherein
R^{P1} and R^{P2} are independently selected from C₁-C₆-alkyl, C₃-C₆-cycloalkyl, phenyl, tolyl and phenoxy;
R^{P3} is selected from phenoxy or biphenyl of formula (L1) 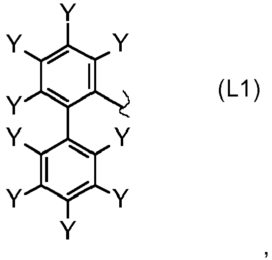 wherein
   - Y: are independently selected from H, C₁-C₆-alkyl, C₁-C₆-haloalkyl, hydroxy, C₁-C₆-alkoxy, phenoxy, phenyl, halo, SO₃H, amino, C₁-C₆-alkylamino or bis(C₁-C₆-alkyl)amino.

R^{P1} of the ligands of formula (L) is selected from C₁-C₆-alkyl, C₃-C₆-cycloalkyl, phenyl, tolyl and phenoxy; preferably from C₃-C₆-cycloalkyl and phenoxy.

According to one embodiment, R^{P1} is C₁-C₆-alkyl, preferably i-Pr or t-butyl.

According to another embodiment, R^{P1} is C₃-C₆-cycloalkyl, preferably cyclohexyl.

According to another embodiment, R^{P1} is phenyl.

According to another embodiment, R^{P1} is tolyl.

According to another embodiment, R^{P1} is phenoxy.

R^{P2} of the ligands of formula (L) is selected from C₁-C₆-alkyl, C₃-C₆-cycloalkyl, phenyl, and phenoxy; preferably from C₃-C₆-cycloalkyl and phenoxy.

According to one embodiment, R^{P2} is C₁-C₆-alkyl, preferably iPr or t-butyl.

According to another embodiment, R^{P2} is C₃-C₆-cycloalkyl, preferably cyclohexyl.

According to another embodiment, R^{P2} is phenyl.

According to another embodiment, R^{P2} is tolyl.

According to another embodiment, R^{P2} is phenoxy.

R^{P3} of the ligands of formula (L) is selected from tolyl, phenoxy or biphenyl of formula (L1)

According to one embodiment, R^{P3} is phenoxy.

According to one embodiment, R^{P3} is tolyl.

According to another embodiment, R^{P3} is biphenyl of formula (L1) 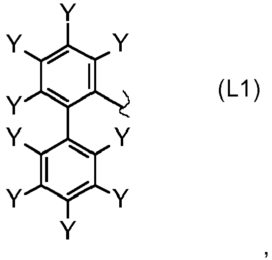 wherein
- Y: are independently selected from H, C₁-C₆-alkyl, C₁-C₆-haloalkyl, hydroxy, C₁-C₆-alkoxy, phenoxy, phenyl, halo, SO₃H, amino, C₁-C₆-alkylamino or bis(C₁-C₆-alkyl)amino; preferably from H, C₁-C₆-alkyl, C₁-C₆-alkoxy, SO₃H, amino, C₁-C₆-alkylamino or bis(C₁-C₆-alkyl)amino; more preferably from H, C₁-C₆-alkyl, C₁-C₆-alkoxy or SO₃H.

According to one specific embodiment, both R^{P1} and R^{P2} are C₁-C₆-alkyl, preferably t-butyl.

According to another specific embodiment, both R^{P1} and R^{P2} are C₃-C₆-cycloalkyl, preferably cyclohexyl.

According to another specific embodiment, both R^{P1} and R^{P2} are phenyl.

According to another specific embodiment, both R^{P1} and R^{P2} are tolyl.

According to another specific embodiment, both R^{P1} and R^{P2} are phenoxy.

Particularly preferred ligands L are given in the Table L below. Each line of said table corresponds to one single embodiment. In said table and anywhere below or above "Me" stands for methyl, "i-Pr" stands for iso-propyl, "t-Bu" stands for tert-butyl, "Ph" stands for phenyl, "Cy" stands for cyclohexyl, "o-Tol" stands for o-Tolyl.

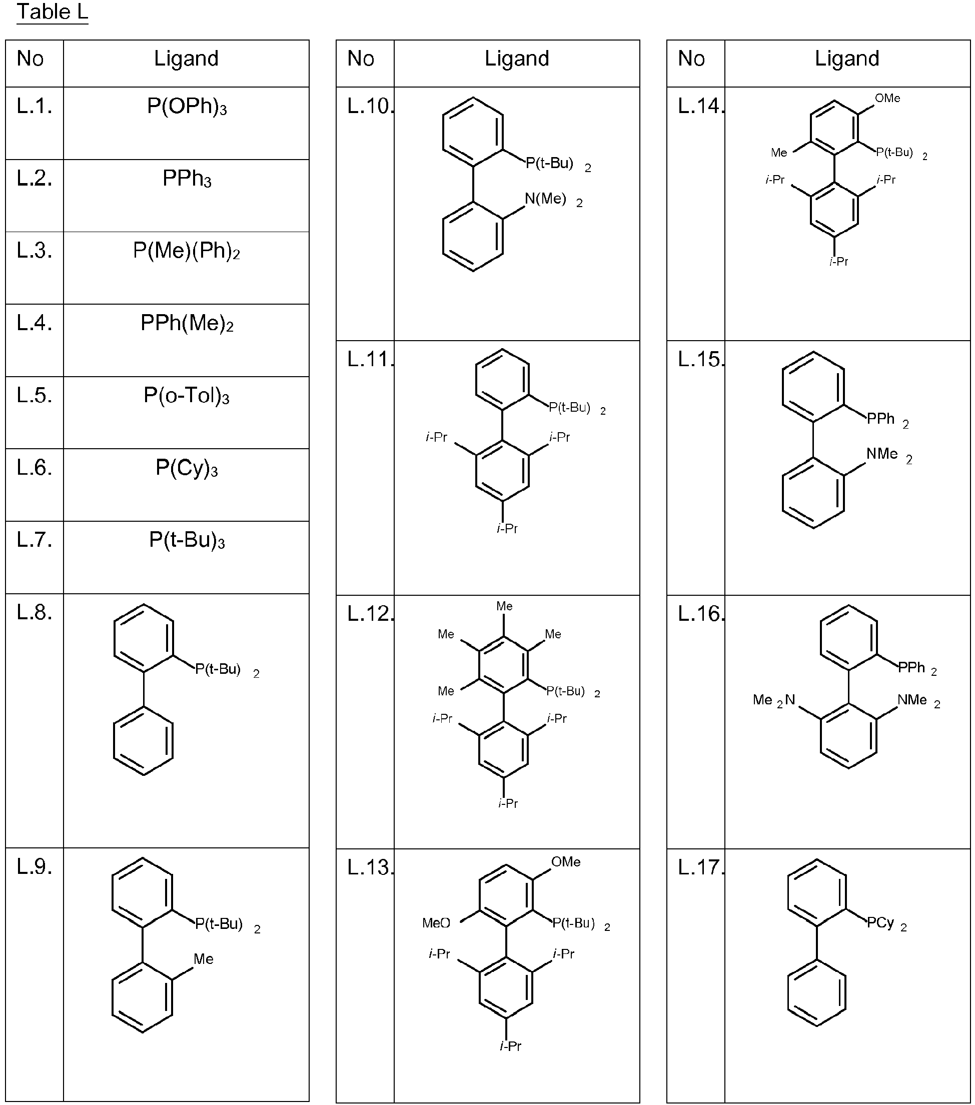

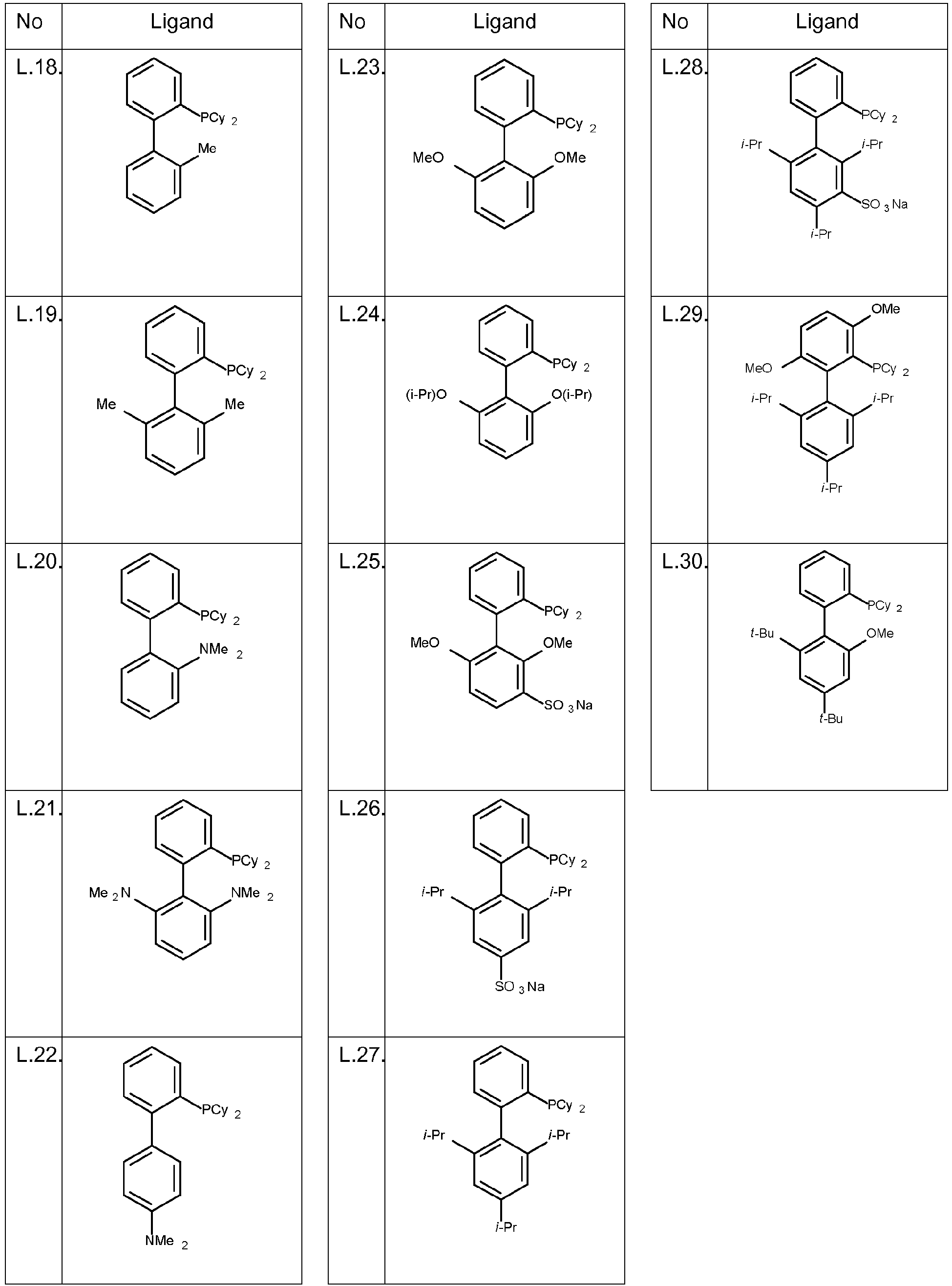

The most preferred ligands are L.1, L11, L.13, L.23, L.27 and L.28.

The preferred combinations of the Pd source and the ligand are given in Table 2.

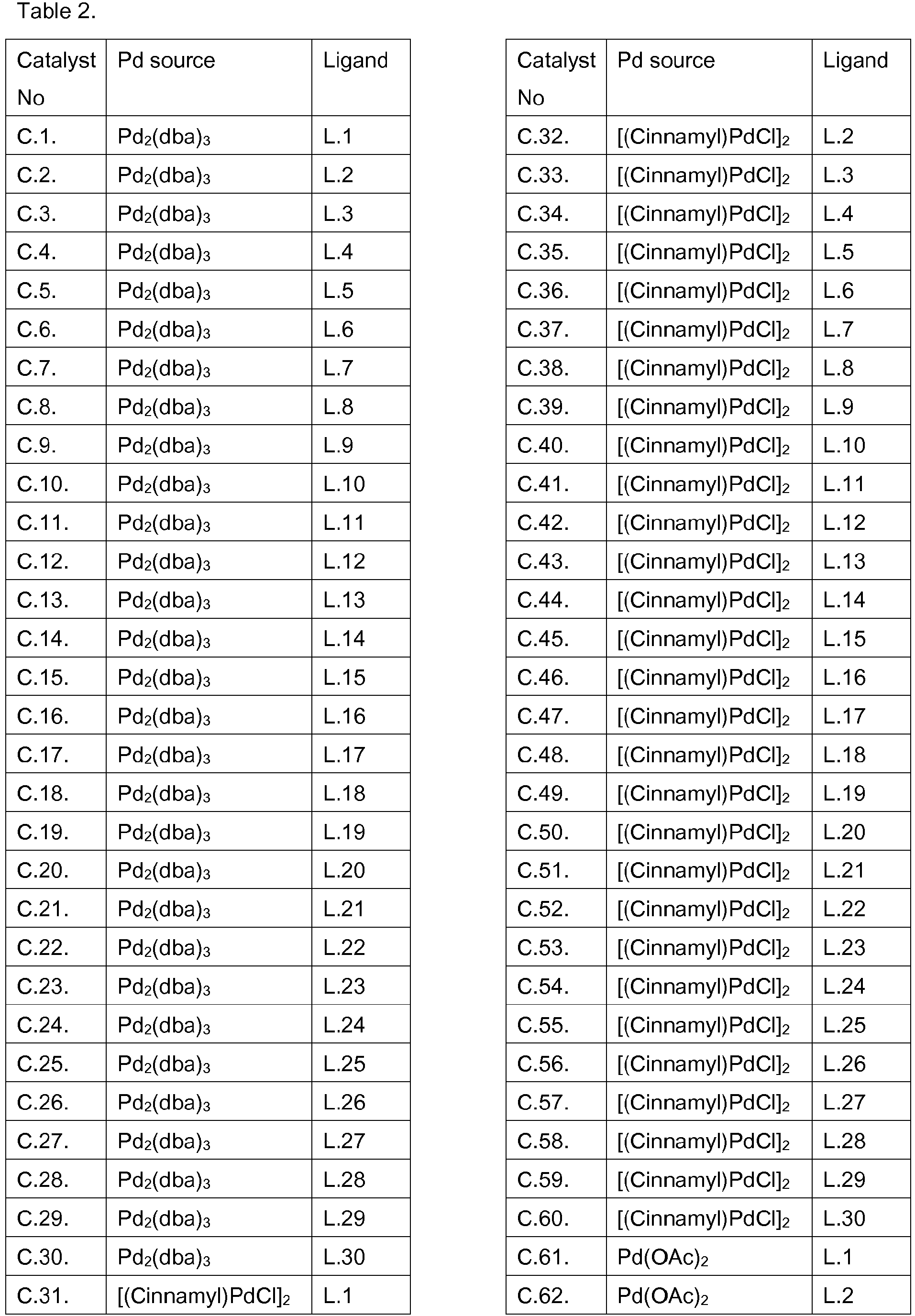

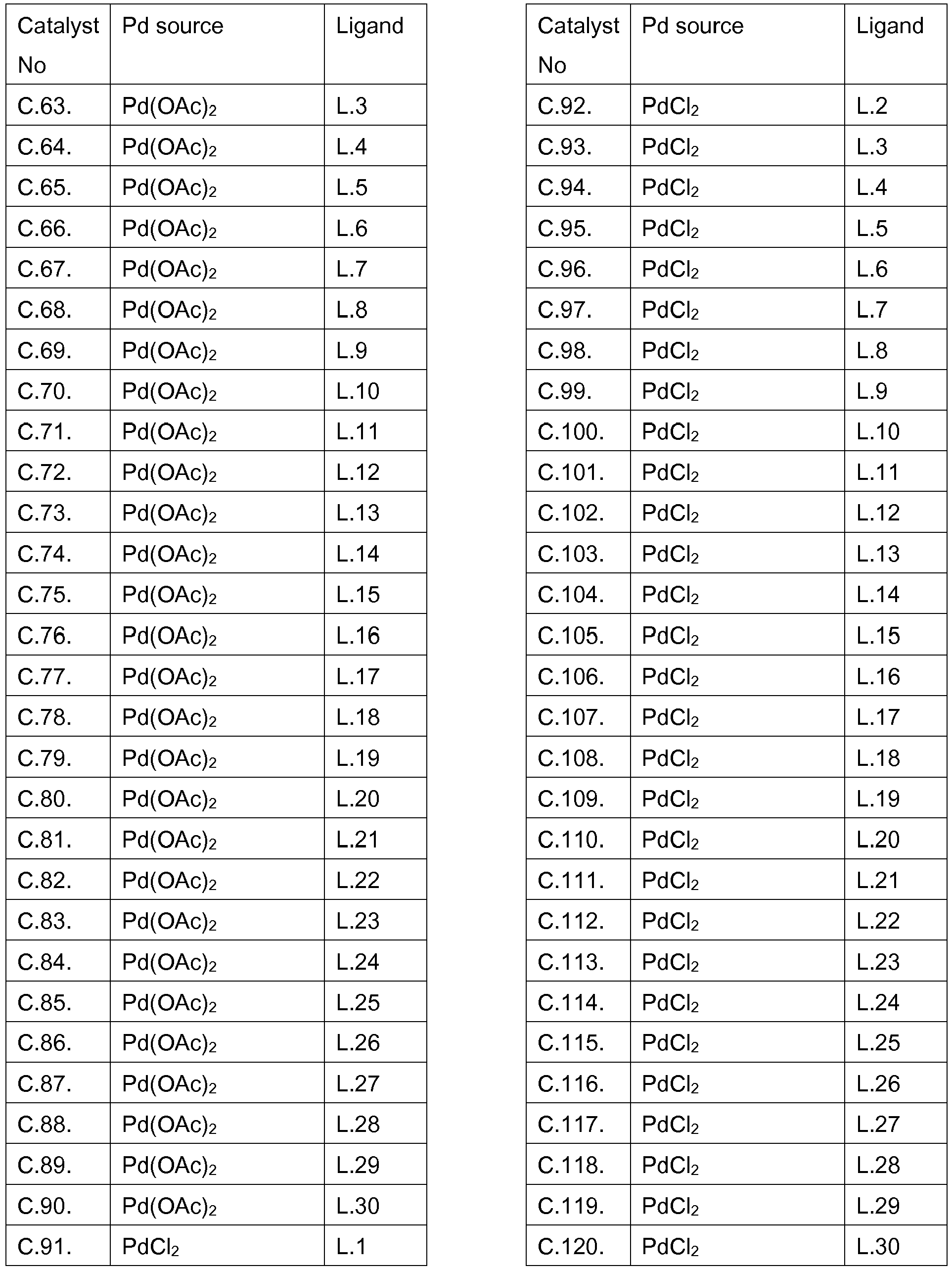

According to another specific embodiment, the reaction of step (i) is carried out in the presence of a precatalyst, in particular of a Pd precatalyst. The precatalyst is preferably selected from the Pd precatalysts of the 1^{st}, 2^{nd}, 3^{rd} or 4^{th} generation.

The precatalysts of the 1^{st} generation have the formula (PC1): 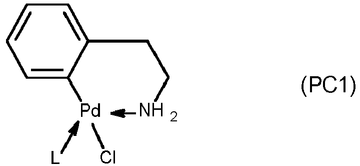 wherein L is a ligand as defined above for the Pd catalysts.

The precatalysts of the 2^{nd} generation have the formula (PC2): 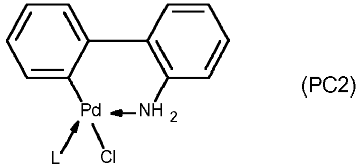 wherein L is a ligand as defined above for the Pd catalysts.

The precatalysts of the 3^{rd} generation have the formula (PC3): 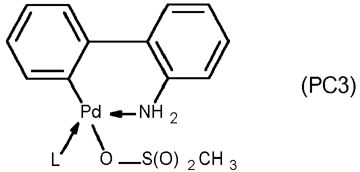 wherein L is a ligand as defined above for the Pd catalysts.

The precatalysts of the 4^{th} generation have the formula (PC4): 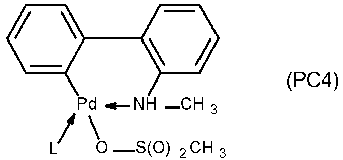 wherein L is a ligand as defined above for the Pd catalysts.

According to one specific embodiment, the precatalyst is the Pd precatalyst of the 1^{st} generation.

According to another specific embodiment, the precatalyst is the Pd precatalyst of the 2^{nd} generation.

According to another specific embodiment, the precatalyst is the Pd precatalyst of the 3^{rd} generation.

According to another specific embodiment, the precatalyst is the Pd precatalyst of the 4^{th} generation.

According to another embodiment, the catalyst is a Cu(I) catalyst, preferably CuCl, CuBr or Cul.

According to another embodiment, the catalyst is a Cu(II) catalyst, preferably CuCl₂, CuBr₂ or Cu(OAc)₂.

According to another embodiment, the catalyst is a Ni catalyst, preferably NiCl₂, NiBr₂, nickel(II) acetylacetonate (Ni(acac)₂), Ni(OAc)₂ or bis(1,5-cyclooctadiene)nickel(0) (Ni(COD)₂).

The catalyst or precatalyst can be added to the reaction mixture in one portion or in several portions. According to one embodiment it is added to the reaction mixture in one portion. According to another embodiment it is added to the reaction mixture in several portions.

The Pd catalyst (calculated as Pd source) is usually used in an amout from 0.0001 to 0.1 mole equivalents per 1 mole of compound (II), preferably from 0.0005 to 0.01 mole equivalents per 1 mole of compound (II), more preferably from 0.001 to 0.01 mole equivalents per 1 mole of compound (II), most preferably from 0.001 to 0.005 mole equivalents per 1 mole of compound (II).

According one embodiment of the invention the cyanide is used in form of an inorganic salt and the catalyst is a pallcadium complex with phosphorous containing ligands as described herein.

According to one specific embodiment, the inorganic salt is KCN and the catalyst is a pallcadium complex with phosphorous containing ligands as described herein.

According to another specific embodiment, the inorganic salt is NaCN and the catalyst is a pallcadium complex with phosphorous containing ligands as described herein.

According to another specific embodiment, the inorganic salt is CuCN and the catalyst is a pallcadium complex with phosphorous containing ligands as described herein.

According to another specific embodiment, the inorganic salt is Ca(CN)₂ and the catalyst is a pallcadium complex with phosphorous containing ligands as described herein.

According to another specific embodiment, the inorganic salt is Zn(CN)₂ and the catalyst is a pallcadium complex with phosphorous containing ligands as described herein.

According to another specific embodiment, the inorganic salt is Co(CN)₂ and the catalyst is a pallcadium complex with phosphorous containing ligands as described herein.

According to one specific embodiment, the inorganic salt is KCN and the catalyst is a Cu(I) catalyst, preferably CuCl, CuBr or CuI, more preferably CuBr.

According to another specific embodiment, the inorganic salt is NaCN and the catalyst is Cu(I) catalyst, preferably CuCl, CuBr or CuI, more preferably CuBr.

According to another specific embodiment, the inorganic salt is CuCN and the catalyst is Cu(I) catalyst, preferably CuCl, CuBr or CuI, more preferably CuBr.

According to another specific embodiment, the inorganic salt is Ca(CN)₂ and the catalyst is Cu(I) catalyst, preferably CuCl, CuBr or CuI, more preferably CuBr.

According to another specific embodiment, the inorganic salt is Zn(CN)₂ and the catalyst is Cu(I) catalyst, preferably CuCl, CuBr or CuI, more preferably CuBr.
According to another specific embodiment, the inorganic salt is Co(CN)₂ and the catalyst is a Cu(I) catalyst, preferably CuCl, CuBr or CuI, more preferably CuBr.
According to one specific embodiment, the inorganic salt is KCN and the catalyst is a Cu(II) catalyst, preferably CuCl₂, CuBr₂ or Cu(OAc)₂, more preferably CuBr₂.
According to another specific embodiment, the inorganic salt is NaCN and the catalyst is a Cu(II) catalyst, preferably CuCl₂, CuBr₂ or Cu(OAc)₂, more preferably CuBr₂.
According to another specific embodiment, the inorganic salt is CuCN and the catalyst is a Cu(II) catalyst, preferably CuCl₂, CuBr₂ or Cu(OAc)₂, more preferably CuBr₂.
According to another specific embodiment, the inorganic salt is Ca(CN)₂ and the catalyst is a Cu(II) catalyst, preferably CuCl₂, CuBr₂ or Cu(OAc)₂, more preferably CuBr₂.
According to another specific embodiment, the inorganic salt is Zn(CN)₂ and the catalyst is a Cu(II) catalyst, preferably CuCl₂, CuBr₂ or Cu(OAc)₂, more preferably CuBr₂.
According to another specific embodiment, the inorganic salt is Co(CN)₂ and the catalyst is a Cu(II) catalyst, preferably CuCl₂, CuBr₂ or Cu(OAc)₂, more preferably CuBr₂.

According another embodiment of the invention the cyanide is used in form of a cyano-metal complex and the catalyst is a pallcadium complex with phosphorous containing ligands as described herein.
According to one specific embodiment, the cyano-metal complex is K₄[Fe(CN)₆] and the catalyst is a pallcadium complex with phosphorous containing ligands as described herein.
According to another specific embodiment, the cyano-metal complex is K₃[Fe(CN)₆] and the catalyst is a pallcadium complex with phosphorous containing ligands as described herein.
According to another specific embodiment, the cyano-metal complex is Na₄[Fe(CN)₆] and the catalyst is a pallcadium complex with phosphorous containing ligands as described herein.
According to another specific embodiment, the cyano-metal complex is Na₃[Fe(CN)₆] and the catalyst is a pallcadium complex with phosphorous containing ligands as described herein.

According another embodiment of the invention the cyanide is used in form of an organic cyanohydrin of formula (CH) and the catalyst is a pallcadium complex with phosphorous containing ligands as described herein. Examples of the suitable cyanohydrins are acetone cyanhydrin or acetophenone cyanhydrin.

The preferred combinatons of the cyanide and the catalyst are given in tables A to L.
Table A. The combinations of the cyanide and the catalyst, wherein the cyanide is KCN and the catalyst for each individual combination corresponds in each case to one line of Table 2 (combinations A.1. to A.120).
Table B. The combinations of the cyanide and the catalyst, wherein the cyanide is NaCN and the catalyst for each individual combination corresponds in each case to one line of Table 2 (combinations B.1. to B.120).
Table C. The combinations of the cyanide and the catalyst, wherein the cyanide is CuCN and the catalyst for each individual combination corresponds in each case to one line of Table 2 (combinations C.1. to C.120).
Table D. The combinations of the cyanide and the catalyst, wherein the cyanide is Ca(CN)₂ and the catalyst for each individual combination corresponds in each case to one line of Table 2 (combinations D.1. to D.120).
Table E. The combinations of the cyanide and the catalyst, wherein the cyanide is Zn(CN)₂ and the catalyst for each individual combination corresponds in each case to one line of Table 2 (combinations E.1. to E.120).
Table F. The combinations of the cyanide and the catalyst, wherein the cyanide is Co(CN)₂ and the catalyst for each individual combination corresponds in each case to one line of Table 2 (combinations F.1. to F.120).
Table G. The combinations of the cyanide and the catalyst, wherein the cyanide is K₄[Fe(CN)₆] and the catalyst for each individual combination corresponds in each case to one line of Table 2 (combinations G.1. to G.120).
Table H. The combinations of the cyanide and the catalyst, wherein the cyanide is K₃[Fe(CN)₆] and the catalyst for each individual combination corresponds in each case to one line of Table 2 (combinations H.1. to H.120).
Table I. The combinations of the cyanide and the catalyst, wherein the cyanide is Na₄[Fe(CN)₆] and the catalyst for each individual combination corresponds in each case to one line of Table 2 (combinations G.1. to G.120).
Table J. The combinations of the cyanide and the catalyst, wherein the cyanide is Na₃[Fe(CN)₆] and the catalyst for each individual combination corresponds in each case to one line of Table 2 (combinations H.1. to H.120).
Table K. The combinations of the cyanide and the catalyst, wherein the cyanide is acetone cyanhydrin and the catalyst for each individual combination corresponds in each case to one line of Table 2 (combinations K.1. to K.120).
Table L. The combinations of the cyanide and the catalyst, wherein the cyanide is acetophenone cyanhydrin and the catalyst for each individual combination corresponds in each case to one line of Table 2 (combinations L.1. to L.120).

The step (i) according to the present invention is carried out in an inert solvent. Examples of suitable solvents are aromatic hydrocarbons, such as benzene, toluene, ethylbenzene, cymene, mesitylene, o-xylene, m-xylene or p-xylene; N-nethyl-2-pyrrolidon (NMP), dimethylformamide (DMF), tetrahydrofurane (THF), 1-4 dioxane, ethanol, 1-propanol, 2-propanol, 1-butanol, 2-butanol, dimethylsulfoxid (DMSO), dimethylacetamid (DMAC) or any mixture thereof. The solvent may contain up to 5 % water. Preferably, the solvent is selected from toluene, NMP, DMF or any mixtures thereof. Most preferred is the mixture of toluene and NMP.

The step (i) according to the process of the present invention can be carried out in the presence of a base. The base used in step (i) can be an inorganic base or an organic base.

According to one embodiment the base is an inorganic base. Suitable inorganic bases are hydroxides, carbonates, hydrocarbonates, phosphates and hydrophosphates of alkali or earth alkali metals or any mixtures thereof. Examples of the inorganic base are NaOH, KOH, Na₂CO₃, K₂CO₃, NaHCO₃, KHCO₃, Na₃PO₄, K₃PO₄, Na₂HPO₄, K₂HPO₄, NaH₂PO₄, KH₂PO₄.

According to one specific embodiment, the base is KOH.

According to another specific embodiment, the base is NaOH.

According to another specific embodiment, the base is K₂CO₃.

According to another specific embodiment, the base is Na₂CO₃.

According to another specific embodiment, the base is NaHCO₃.

According to another specific embodiment, the base is KHCO₃.

According to another specific embodiment, the base is Na₃PO₄.

According to another specific embodiment, the base is K₃PO₄.

According to another specific embodiment, the base is Na₂HPO₄.

According to another specific embodiment, the base is K₂HPO₄.

According to another specific embodiment, the base is NaH₂PO₄.

According to another specific embodiment, the base is KH₂PO₄.

The base can be used in a solid form or as a solution, e.g. as aqueous solution.

According to one embodiment, if X in compound (II) is Br, the inorganic salt is not CuCN.

According to another embodiment the base is an organic base. Examples of suitable organic bases are tertiary amines, pyridine, substituted pyridines, amidines, bicyclic amines or any mixture thereof.

Examples of suitable tertiary amines are tri-(C₁-C₆)-alkylamines such as trimethylamine, triethylamine, tributylamine and N,N-diisopropylethylamine; di-(C₁-C₆)-alkyl-phenylamines such as N,N-dimethylaniline and N,N-diethylaniline; N-methyl imidazole, N,N-dimethylaminopyridine and the like.

Examples of suitable substituted pyridines are collidine, lutidines, picolines, N,N-dimethylaminopyridine and the like.

According to another specific embodiment the organic base is selected from amidines. Examples of suitable amidines are 1,8-diazabicyclo[5.4.0]undec-7-en (DBU) and 1,5-diazabicyclo-[4.3.0]non-5-ene (DBN).

Examples of suitable bicyclic amines are 1,4-diazabicyclo[2.2.2]octane (DABCO) and the like.

The molar ratio of the base to compound (II) is generally in the range from 4:1 to 0.2:1, preferably from 3:1 to 0.5:1, more preferably from 2:1 to 0.8:1, most preferably from 1.5:1 to 1:1.

The reagents for step (i) are preferably added at ambient temperature and the reaction temperature is then elevated, wherein the reaction temperature after the reagents have been added is preferably held at a maximum of 165°C, more preferably at a maximum of 155°C, most preferably at a maximum of 145°C. Generally, it is preferred to have a reaction temperature at a minimum of 60°C, more preferably at a minimum of 70°C, most preferablyat a minimum of 80°C. The preferred temperature range is from 60 to 165°C, more preferred from 70°C to 155°C, most prefereed from 80°C to 145°C.

According to step (ii), the compound (I) is reacted with a phenol of formula (III)

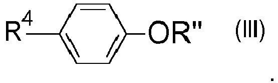

R" in fomula (III) is hydrogen or an alkali metal kation.

R⁴ in formula (III) is halogen, preferably F or Cl, more preferably Cl.

Compound (I) is commercially available or can be synthesized as described below or as known in the art.

Compounds (III) can be synthesized as known to the skilled person or are also commercially available.

If R" in fomula (III) is hydrogen, the reaction is carried out in the presence of a base.

The base used in step (ii) is preferably an inorganic base. Suitable inorganic bases are hydroxides, carbonates or hydrocarbonates of alkali or earth alkali metals; amine bases or any mixtures thereof. The inorganic base is preferably selected from NaOH, KOH, Na₂CO₃, K₂CO₃, Ca-CO₃ and NH₃, more preferably from Na₂CO₃ and K₂CO₃. According to one particular embodiment, Na₂CO₃ is used. According to a further particular embodiment, K₂CO₃ is used.

The base can be used in a solid form, as a solution, e.g. as aqueous solution, or in a gaseous form.

The base used in step (ii) can also be an organic base. The organic base is preferably selected from amine bases such as, tertiary amines, quaternary ammonium salts, amidines, guanidine derivatives, pyridine, substituted pyridines, bicyclic amines or any mixture thereof.

According to one specific embodiment the organic base is selected from tertiary amines. Examples of suitable tertiary amines are tri-(C₁-C₆)-alkylamines such as trimethylamine, triethylamine, tributylamine and N,N-diisopropylethylamine; di-(C₁-C₆)-alkyl-phenylamines such as N,N-dimethylaniline and N,N-diethylaniline; N-methyl imidazole, N,N-dimethylaminopyridine and the like. Preferable tertiary amine bases are selected from trimethylamine, triethylamine, tributylamine and N,N-diisopropylethylamine.

According to another specific embodiment the organic base is selected from quaternary ammonium salts. Example of a suitable quaternary ammonium salt is tetrabutylammonium fluoride (TBAF).

According to another specific embodiment the organic base is selected from amidines. Examples of suitable amidines are 1,8-diazabicyclo[5.4.0]undec-7-en (DBU) and 1,5-diazabicyclo-[4.3.0]non-5-ene (DBN).

According to another specific embodiment the organic base is selected from guanidine derivatives. Examples of suitable guanidine derivatives tetramethyl guanidine (TMG), 1,5,7-triazabicyclo[4.4.0]dec-5-ene (TBD) and the like.

According to another specific embodiment the organic base is selected from pyridine and substituted pyridines. Pyridine is preferred. Examples of suitable substituted pyridines are collidine, lutidines, picolines, N,N-dimethylaminopyridine and the like.

According to another specific embodiment the organic base is selected from bicyclic amines. Example of suitable bicyclic amines is 1,4-diazabicyclo[2.2.2]octane (DABCO) and the like.

The molar ratio of the base to compound (I) is generally in the range from 0.5:1 to 4:1, preferably from 0.5:1 to 3.5:1, more preferably from 0.8:1 to 2:1, most preferably from 0.8:1 to 1.5:1.

The process step (ii) according to the present invention can be carried out in the presence of a catalyst. The catalyst is preferably a Cu or Pd catalyst or Bu₄NBr.

According to one embodiment, the catalyst is a Cu catalyst. Preferably, the Cu catalyst is selected from Cu, Cu(OAc)₂, CuI, CuO, CuCl₂ or Cu(acac)₂ (Copper(II) acetylacetonate).

According to another embodiment, the catalyst is a Pd catalyst. Preferably, the Pd catalyst is selected from Pd/C and PdCl₂.

According to another embodiment, the catalyst is Bu₄NBr.

The molar ratio of the catalyst to compound (I) is generally in the range from 0.001:1 to 0.5:1, preferably from 0.005:1 to 0.2:1, more preferably from 0.01:1 to 0.1:1, most preferably from 0.01:1 to 0.05:1.

Examples for appropriate solvents for step (ii) of the inventive process are aprotic organic solvents such as for example dimethyl formamide (DMF), N-methyl pyrrolidone (NMP), dimethyl imidazolidinone (DMI), toluene, o-xylene, m-xylene, p-xylene, dimethylactamide (DMA), DMSO and any mixtures thereof. In particular DMF, NMP, toluene and DMA or any mixtures thereof, more specifically DMF, are particularly suitable.

According to one specific embodiment, the solvent used in step (ii) contains not more than 3 eq DMF in relation to 1 eq of the phenol of formula (III), preferably not more than 2.8 eq to 1 eq of the phenol of formula (III), more preferably not more than 2.6 eq to 1 eq of the phenol of formula (III), most preferably not more than 2.4 eq to 1 eq of the phenol of formula (III), specifically not more than 2.2 eq DMF to 1 eq of the phenol of formula (III).

The reagents for step (ii) are preferably added at ambient temperature and the reaction temperature is then elevated, wherein the reaction temperature after the reagents have been added is preferably held at a maximum of 150°C, in particular at a maximum of 140°C, more preferably at a maximum of 130°C. Generally, it is preferred to have a reaction temperature of 20°C to 135°C, in particular 50°C to 135°C, more particularly 100°C to 130°C.

After step (ii), a work-up of the reaction mixture can be carried out by procedures known in a general manner to the person skilled in the art. Generally, water is added and the aqueous phase is extracted with a suitable solvent, e.g. tolene or o-xylene. The raw product obtained after evaporation of the solvent(s) can directly be used in a further step, if desired. However, the raw product can also be further worked up and/or purified as generally known to the skilled person.

According to one embodiment of the invention, after completion of the reaction, most of the solvent (e.g. DMF or toluene) is removed from the reaction mixture, preferably under reduced pressure. Then, a suitable organic solvent, such as, for example, toluene or o-xylene, is added together with water. According to the inventive process, it may be favorable to carry out one to three, preferably two extractions of the aqueous phase.

The ketones (V) obtained according to the inventive process can be used as reagent for the synthesis of an oxirane of the formula (O) that are useful intermediates for the synthesis of triazole active ingredients of formula (T), that are effective against phytopathogenic fungi.

In the following, a possible synthesis route to such fungicides using said intermediates (V) is described:
Consequently, the present invention also relates to a process for the preparation of triazole compounds of the formula (T) 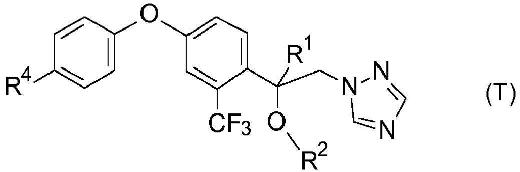 wherein
R¹ is C₁-C₆-alkyl or C₃-C₈-cycloalkyl;
R² is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl; wherein the aliphatic moieties of R² are not further substituted or do carry one, two, three or up to the maximum possible number of identical or different groups R^{2a} which independently are selected from:
   R^{2a} halogen, OH, CN, nitro, C₁-C₄-alkoxy, C₃-C₈-cycloalkyl, C₃-C₈-halocycloalkyl and C₁-C₄-halogenalkoxy;
wherein the cycloalkyl and/or phenyl moieties of R² are not further substituted or do carry one, two, three, four, five or up to the maximum number of identical or different groups R^{2b} which independently are selected from:
   R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
R⁴ is halogen, preferably F or Cl;
comprising the following steps:
(iii) as described herein to obtain the compound (V);
(iv) reacting the ketone of the formula (V) 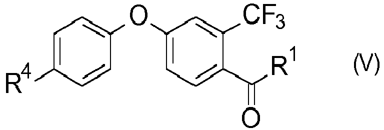 wherein the variables have the following meaning:
   - R¹: is C₁-C₆-alkyl or C₃-C₈-cycloalkyl;
   - R⁴: is as defined for compounds of formula (T);
   to oxiranes (O); 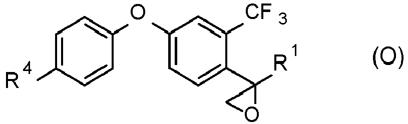 wherein
   R¹ and R⁴ are as defined for compounds of formula (T);
(v) reacting the oxirane (O) with 1H-1,2,4-triazole in the presence of a base to obtain compounds (T), wherein R² is hydrogen (compounds T-1);
and, for obtaining compounds wherein R² is different from hydrogen:
b) derivatizing the compound of formula (T-1) of step (iii) under basic conditions with R²-LG, wherein LG is a nucleophilically replaceable leaving group; to result in compounds (T-2).

According to one embodiment, R² is selected from C₁-C₆-alkyl, C₂-C₆-alkenyl, C₂-C₆-alkynyl, C₃-C₈-cycloalkyl, C₃-C₈-cycloalkyl-C₁-C₄-alkyl, phenyl, phenyl-C₁-C₄-alkyl, phenyl-C₂-C₄-alkenyl and phenyl-C₂-C₄-alkynyl, wherein the R² are in each case unsubstituted or are substituted by R^{12a} and/or R^{12b} as defined and preferably defined herein.

According to a further embodiment, R² is C₁-C₆-alkyl, in particular C₁-C₄-alkyl, such as CH₃, C₂H₅, CH(CH₃)₂, CH₂CH₂CH₃, CH₂CH₂CH₂CH₃, CH₂CH(CH₃)₂. A further embodiment relates to compounds, wherein R² is C₁-C₆-alkyl, in particular C₁-C₄-alkyl, that is substituted by one, two or three or up to the maximum possible number of identical or different groups R^{12a}, as defined and preferably defined herein. According to still another embodiment, R² is C₃-C₈-cycloalkyl-C₁-C₆-alkyl, in particular C₃-C₆-cycloalkyl-C₁-C₄-alkyl. A further embodiment relates to compounds, wherein R² is C₃-C₈-cycloalkyl-C₁-C₆-alkyl, in particular C₃-C₆-cycloalkyl-C₁-C₄-alkyl, more particularly C₃-C₆-cycloalkyl-C₁-C₂-alkyl, that is substituted by one, two or three or up to the maximum possible number of identical or different groups R^{12a} in the alkyl moiety and/or substituted by one, two, three four or five or up to the maximum possible number of identical or different groups R^{12b} in the cycloalkyl moiety. R^{12a} and R^{12b} are in each case as defined and preferably defined herein.

According to another embodiment, R² is C₂-C₆-alkenyl, in particular C₂-C₄-alkenyl, such as CH₂CH=CH₂, CH₂C(CH₃)=CH₂ or CH₂CH=CHCH₃. A further embodiment relates to compounds, wherein R² is C₂-C₆-alkenyl, in particular C₂-C₄-alkenyl, that is substituted by one, two or three or up to the maximum possible number of identical or different groups R^{12a} as defined and preferably defined herein. According to a specific embodiment thereof, R² is C₂-C₆-haloalkenyl, in particular C₂-C₄-haloalkenyl, such as CH₂C(Cl)=CH₂ and CH₂C(H)=CHCl. According to still another embodiment, R² is C₂-C₆-alkynyl, in particular C₂-C₄-alkynyl, such as CH₂C≡CH or CH₂C≡CCH₃. A further embodiment relates to compounds, wherein R² is C₂-C₆-alkynyl, in particular C₂-C₄-alkynyl, that is substituted by one, two or three or up to the maximum possible number of identical or different groups R^{12a}, as defined and preferably defined herein.

According to still another embodiment, R² is C₃-C₈-cycloalkyl, in particular C₃-C₆-cycloalkyl, such as C₃H₅ (cyclopropyl), C₄H₇ (cyclobutyl), cyclopentyl or cyclohexyl. A further embodiment relates to compounds, wherein R² is C₃-C₈-cycloalkyl, in particular C₃-C₆-cycloalkyl, such as C₃H₅ (cyclopropyl) or C₄H₇ (cyclobutyl), that is substituted by one, two, three four or five or up to the maximum possible number of identical or different groups R^{12b} as defined and preferably defined herein. In a further embodiment of the invention, R² is selected from C₁-C₆-alkyl, C₂-C₆-alkenyl and C₂-C₆-alkynyl, wherein the R² are in each case unsubstituted or are substituted by R^{12a} and/or R^{12b} as defined and preferably defined herein. In each case, the substituents may also have the preferred meanings for the respective substituent as defined above.

R^{12a} according to the invention is preferably independently selected from F, Cl, OH, CN, C₁-C₂-alkoxy, cyclopropyl, 1-F-cyclopropyl, 1-Cl-cyclopropyl and C₁-C₂-halogenalkoxy.

R^{12b} according to the invention is preferably independently selected from F, Cl, OH, CN, nitro, CH₃, OCH₃, cyclopropyl, 1-F-cyclopropyl, 1-Cl-cyclopropyl and halogenmethoxy.

Specifically, the following compounds T.1 to T.7 can advantageously be prepared using the process according to the present invention:

| | |
|---|---|
| compound T.1 | 2-[4-(4-chlorophenoxy)-2-(trifluoromethyl)phenyl]-1-(1,2,4-triazol-1-yl)propan-2-ol; |
| compound T.2 | 1-[4-(4-chlorophenoxy)-2-(trifluoromethyl)phenyl]-1-cyclopropyl-2-(1,2,4-triazol-1-yl)ethanol; |
| compound T.3 | 2-[4-(4-chlorophenoxy)-2-(trifluoromethyl)phenyl]-3-methyl-1-(1,2,4-triazol-1-yl)butan-2-ol; |
| compound T.4 | 2-[4-(4-chlorophenoxy)-2-(trifluoromethyl)phenyl]-1-(1,2,4-triazol-1-yl)butan-2-ol; |
| compound T.5 | 1-[2-[4-(4-chlorophenoxy)-2-(trifluoromethyl)phenyl]-2-methoxy-propyl]-1,2,4-triazole; |
| compound T.6 | 1-[2-[4-(4-chlorophenoxy)-2-(trifluoromethyl)phenyl]-2-cyclopropyl-2-methoxyethyl]-1,2,4-triazole; |
| compound T.7 | 1-[2-[4-(4-chlorophenoxy)-2-(trifluoromethyl)phenyl]-2-methoxy-butyl]1,2,4-triazole; |

Compounds (T) comprise chiral centers and they are generally obtained in the form of racemates. The R- and S-enantiomers of the compounds can be separated and isolated in pure form with methods known by the skilled person, e.g. by using chiral HPLC. Furthermore, components I can be present in different crystal modifications, which may differ in biological activity. The compounds may be present in various crystal modifications. They are likewise provided by the present invention.

In process step (iv), for obtaining an oxirane from the keto group, compound (V) is preferably reacted with a trimethylsulf(ox)onium halide (CH₃)₃S⁺ (O)Hal⁻ (S1) or trimethylsulfonium methylsulfate (CH₃)₃S⁺ CH₃SO₄⁻ (S2).

According to one embodiment, in the process step (iv), the ketone (V) is reacted with trimethylsulfonium methylsulfate of the formula (S2) (CH₃)₃S⁺ CH₃SO₄⁻, preferably in aqueous solution in the presence of a base.

Step (iv) for the preparation of oxiranes (O) particularly is as follows:
(iv) reacting an oxo compound of the formula (V) with trimethylsulfonium methylsulfate of the formula (S2)

S2 (CH₃)₃S⁺ CH₃SO₄⁻

in aqueous solution in the presence of a base, wherein the variables R¹, R⁴ are defined as given and preferably described herein for compounds (T).

In this process step (iv) using trimethylsulfonium methylsulfate of the formula (S2), preferably, 1 to 4 equivalents, in particular 1.2 to 3.5 eq, more specifically 1.5 to 3.3 eq, of water in relation to one equivalent of compound (V) are used. It may be favorable, if more than 1.5 eq of water, in particular more than 1.5 eq of water to 4 eq of water, more specifically more than 1.5 eq to 3.5 eq of water, even more particularly more than 1.5 eq water to 2.5 eq water per mole of compound (V) are used. In particular the ratios of 1.6 to 3.8, more specifically 1.7 to 3.3 eq, more specifically 1.8 to 2.8 eq or 1.9 to 2.5 of water per mole of compound (V) may be favorable according to the present invention. According to a further embodiment, more than 1.5 eq of water, in particular more than 2 eq of water, more specifically more than 2.5 eq of water per mole of compound (V) are used. In particular, 1.6 to 5, more specifically 1.7 to 4 eq, more specifically 1.8 to 3.5 eq of water per mole of compound (V) may be favorable according to the present invention.

The reagent (S2) is preferably used in an amount of 1.1 to 2.5, in particular 1.2 to 2, more specifically 1.3 to 1.6 equivalents of (S2) per 1 equivalent (mole) of compound (V).

In general, the reagent of formula (S2) can be prepared from dimethylsulfide and dimethylsulfate. According to one embodiment, reagent (S2) is prepared in-situ by adding dimethylsulfate to the reaction mixture containing dimethylsulfide. According to a further embodiment, either dimethylsulfide or dimethylsulfate is charged first and the other reagent is then added, wherein it may be preferred to add dimethylsulfide to a reaction mixture containing dimethylsulfate. The dimethylsulfide is usually used in excess. In particular, dimethylsulfide is generally used in amounts so that the reagent (S2) is sufficiently formed during the reaction. The molar ratio between dimethylsulfide and dimethylsulfate for the formation of the reagent (S2) is 1:1 to 2:1. Preferably, the molar ratio between dimethylsulfide and dimethylsulfate is 1:1 to 1.5:1, more preferably 1:1 to 1.4:1. It may be also preferred to use 1 to 1.3, in particular 1 to 1.25, more specifically 1 to 1.1 eq dimethylsulfide in relation to one equivalent of dimethylsulfate.

It is preferred to use as reagent (S2) an aqueous solution of trimethylsulfonium methylsulfate containing 33 to 37 wt%, preferably 34 to 36 wt%, more specifically 34 to 35.3 wt%, also more specifically 34.3 to 35.9 wt%, of trimethylsulfonium kation.

In particular, the reagent (S2) solution contains 33 to 37 wt%, preferably 34 to 36 wt%, more specifically 34 to 35.3 wt%, also more specifically 34.3 to 35.9 wt%, of trimethylsulfonium kation. Accordingly, the amount of trimethylsulfonium-methylsulfate in the reagent, measured as summation of trimethsulfonium-cation and methylsulfate-anion, is about 80 to 90 wt%, preferably about 83 to 88 wt-%, more specifically about 83 to 86 wt-%. The quantification can be, for example, accomplished by means of quantitative NMR-spectroscopie.

The viscosity of the aqueous reagent (S2) solution is comparatively low. The solutions are stable at room temperature, in particular at 25°C, and can be stored over a longer time. In particular, the reagent solution does not crystallize out during storage over a longer time, such as several weeks, e.g. up to 12 weeks, at temperatures of 10 to 25°C.

The reagent can be prepared by adding dimethylsulfate to water and dimethylsulfide. Dimethylsulfide is normally used in excess, generally 2 to 8, more preferably 4 to 6, more specifically 4.5 to 5.5, equivalents.

In the preparation of the aqueous solution of reagent (S2), in particular 0.8 to 2.2 eq, more preferably 0.9 to 1.2 eq, water in relation to the dimethylsulfate are used. It may also be preferred if in the preparation of the aqueous solution of reagent (S2), preferably 1.3 to 2.2 eq, more preferably 1.45 to 2.0 eq, water in relation to the dimethylsulfate are used.

Preferably, the temperature of the reaction mixture when adding the dimethylsulfate is room temperature, in particular 25°C to 40°C.

The aqueous reagent separates as the lower phase and can be further used as such.

The use of the aqueous solution of the reagent (S2) has been proven very efficient also for up-scaled reaction conditions, since it is stable and since it contains a defined amount of reagent, so that reagent (S2) can be easily and precisely dosed to the reaction mixture.

Thus, it is a preferred embodiment, if the reagent (S2) is added as an aqueous solution of trimethylsulfonium methylsulfate containing 33 to 37 wt%, preferably 34 to 36 wt%, more specifically 34 to 35.3 wt%, also more specifically 34.3 to 35.9 wt% of trimethylsulfonium kation.

According to one embodiment of the inventive process, dimethylsulfide is also used as solvent in step (iv). According to a further embodiment, an additional solvent is used. In particular, an aprotic organic solvent is suitable, such as for example diethylether, methyl-tert-butylether, chlorobenzene, xylene or toluene.

The base that can be used in step (iv) is preferably selected from KOH and NaOH. In a preferred embodiment, KOH is used, preferably as solid pellets or flakes. It is preferred if at least 3 equivalents of base, preferably at least 3.2 eq, more specifically at least 3.4 eq per 1 equivalent of compound (V) are used. It may be preferred if the amount of base is 3 to 6 eq, more specifically 3 to 5 eq per mole of compound (V).

The reaction temperature when adding KOH in step (iv) is preferably held at a maximum of 60°C, more specifically at a maximum of 50°C. Generally, it is also preferred to have a reaction temperature when adding KOH of at least 20°C, in particular at least room temperature, in particular at least 25°C. In a further embodiment, the temperature is at least 30°C. It may be preferred if the temperature is at least 35°C or at least 45°C. The temperature of the reaction mixture can for example be held in these ranges by adding the KOH in portions.

The overall reaction temperature in step (iv) is preferably held at a maximum of 70°C, in particular at a maximum of 60°C, more preferably at a maximum of 50°C. Generally, it is also preferred to have a reaction temperature of at least 20°C, in particular at least room temperature, in particular at least 25°C. In a further embodiment, the temperature is at least 30°C. It may be preferred if the temperature is at least 35°C.

In case a work-up of the reaction mixture after step (iv) is suitable, it can be carried out by procedures known in a general manner to the person skilled in the art. It may be preferred if water is added to the reaction mixture after completion of step (iv) and the resulting mixture is heated while stirring dependent on the melting point of the organic content. The temperature during this heating is held preferably from 30°C to 70°C, more specifically 40°C to 60°C, even more specifically 50°C to 60°C. The organic phase may, for example, be separated and dissolved in a suitable solvent such as dimethyl formamide (DMF), N-methylpyrrolidone (NMP), dimethylsulfoxide (DMSO) or dimethylacetamide (DMAC). Dimethylsulfide, if still present, is preferably removed by distillation before or after the solvent addition. The reaction mixture may then be used directly for the next step or, if appropriate, further worked-up and/or purified by e.g. recrystallization and/or chromatography.

According to one further specific embodiment, in step (iv), the oxo compound of the formula (V) is reacted with dimethyl sulfide (CH₃)₂S and dimethylsulfate (CH₃)₂SO₄, forming the reagent (S2), trimethylsulfonium methylsulfate [(CH₃)₃S⁺ CH₃SO₄⁻], in aqueous solution in the presence of potassium hydroxide (KOH), wherein dimethyl sulfide and dimethyl sulfate are used in a molar ratio of 1:1 to 2:1, and wherein at most 10 weight-% organic solvent in relation to the amount of compound (V), are added. For details see also WO2016/005211. In this embodiment, the reagent of formula (S2) is formed from dimethylsulfide and dimethylsulfate. In particular, reagent (S2) is prepared in-situ. Either dimethylsulfide or dimethylsulfate is charged first and the other reagent is then added. It may be preferred to add dimethylsulfide to a reaction mixture containing dimethylsulfate.

The dimethylsulfide and dimethylsulfate are preferably used in such amounts that the reagent (S2) is present in the reaction mixture in an amount of 1.1 to 2.5, in particular 1.2 to 2, more specifically 1.3 to 1.6 equivalents of (S2) per 1 equivalent (mole) of compound (V).

Dimethylsulfide is used in amounts so that the reagent (S2) is sufficiently formed during the reaction. The molar ratio between dimethylsulfide and dimethylsulfate for the formation of the reagent (S2) is 1:1 to 2:1. Preferably, the molar ratio between dimethylsulfide and dimethylsulfate is 1:1 to 1.5:1, more preferably 1:1 to 1.4:1. It may be also preferred to use 1 to 1.3, in particular 1 to 1.25, more specifically 1 to 1.1 dimethylsulfide in relation to one equivalent of dimethylsulfate.

This reaction step can be carried out with at most 10 weight-% of organic solvents in relation to the amount of compound (V) [amount of solvent: (amount of solvent + amount of compound IV)]. In particular, the reaction can be carried out using at most 8 weight-%, more specifically at most 5 weight-%, even more specifically at most 3 weight-%, of organic solvents in relation to the amount of compound (V). More specifically, in the reaction mixture, at most 2 weight-%, more specifically at most 1 weight-% of organic solvents in relation to the amount of compound (V) are added.

In a specific embodiment, in this step (iv) essentially no organic solvent is added. In particular, in step (iv) no organic solvent is added apart from the reagents used.

Organic solvents are liquid organic compounds that dilute the reagents without taking part in the reaction or catalyzing the reaction. The skilled person in the field of organic synthesis is familiar with "organic solvents" and it is clear to such skilled person what kind of solvents are "organic solvents". Examples for organic solvents are e.g. alcohols, nitrils and aromatic hydrocarbons. Alcohols are for example methanol, ethanol, propanol and butanol (e.g. tert-butanol). Aromatic hydrocarbons are for example toluene or xylenes. An example for nitrile is acetonitrile.

Reaction step (iv) is preferably carried out in aqueous solution. Preferably, water is used in an amount of 0.5 to 4 eq, in particular 0.9 to 4, in relation to one equivalent of compound (V). According to one embodiment, relatively low amounts of water, for example 0.5 to 0.95 eq, more specifically 0.6 to 0.94, even more specifically 0.7 to 0.93 eq in relation to one equivalent of compound (V), are used. It may also be advantageous to use 0.8 to 0.92 eq, more specifically 0.85 to 0.91, even more specifically 0.85 to 0.9 eq in relation to one equivalent of compound (V) in the inventive process. According to a further embodiment, 0.9 to 4 equivalents, more specifically 1 to 4, in particular 1.2 to 3.5 eq, more specifically 1.5 to 3.3 eq, of water in relation to one equivalent of compound (V) are used. It may be favorable, if more than 1.5 eq of water, in particular more than 1.5 eq of water to 4 eq of water, more specifically more than 1.5 eq to 3.5 eq of water, even more particularly more than 1.5 eq water to 2.5 eq water per mole of compound (V). In particular the ratios of 1.6 to 3.8, more specifically 1.7 to 3.3 eq, more specifically 1.8 to 2.8 eq or 1.9 to 2.5 of water per mole of compound (V) may be favorable according to the present invention. In one further particular embodiment, advantages can be achieved if the amounts of water used in step (iv) are 0.5 to 0.95 eq or more than 1.5 eq of water to 4 eq per mole of compound (V).

In step (iv), preferably KOH is used. It is preferred if at least 2 equivalents of base, more specifically at least 2.5 equivalents of base, even more specifically at least 3 equivalents of base per 1 equivalent of compound (V) are used. It may be preferably if at least 3.2 eq, more specifically at least 3.4 eq per 1 equivalent of compound (V) are used. Furthermore, it may be advantageous, if the amount of base is 2 to 6 eq, in particular 2.5 to 5.5 eq, more specifically 2.5 to 5 eq, even more specifically 3 to 5 eq per mole of compound (V).

KOH is in particular used in solid form, preferably as solid pellets, flakes, micropills and/or powder.

The base, in particular solid KOH, is in particular used such that the preferred range of water present in the reaction is kept. Then, some of the base is dissolved in the reaction solution and some is still present in solid form during the reaction.

The KOH can be added in one or more portions, for example 2 to 8 portions, to the reaction mixture. KOH can also be added in a continuous manner. Preferably, the KOH is added after compound (V) has been charged to the reaction vessel. However, the order may also be changed and the compound (V) is added to the reaction mixture already containing the KOH.

The order of adding the reagents to the reaction mixture is variable. In one embodiment, the base is added to the solution of compound (V) and solvent first and then reagent (S2) is added. According to another embodiment, the reagent (S2) is added first to the solution of compound (V) and then the base is added. According to a further embodiment, a solution of compound (V) and the reagent (S2) are added simultaneously to the base. In the latter embodiment, the base is preferably suspended in sufficient solvent and is stirred during the addition of the reagents.

In step (v) the oxiranes (O) can be further reacted to a triazole of formula (T) as defined above.

LG represents a nucleophilically replaceable leaving group such as halogen, alkylsulfonyl, alkylsulfonyloxy and arylsulfonyloxy, preferably chloro, bromo or iodo, particularly preferably bromo.

In one embodiment of the invention, in the process step (v) an inorganic base is used.

The inorganic base that may be used in step (v) is preferably selected from NaOH, KOH, Na₂CO₃ and K₂CO₃, more specifically from NaOH and KOH. According to one embodiment, NaOH is used. According to a further embodiment, KOH is used.

According to a specific embodiment, the sodium salt of 1H-1,2,4-triazole as a base is used, wherein said sodium salt is prepared using triazole and a base preferably selected from NaOH, NaH and Na-alcoholates. See also DE 3042302.

The amount of base used in step (v) is preferably equal to or less than 1 eq, in particular less than 1 eq, more preferably equal to or less than 0.8 eq, even more preferably equal to or less than 0.6 equivalents per 1 equivalent of compound (O). Also preferred are amounts of base being equal to or less than 0.4 equivalents, in particular equal to or less than 0.2 equivalents, specifically equal to or less than 0.1 eq per 1 equivalent of compound (O). Preferably, at least 0.1 eq, more preferably at least 0.2 equivalents, in particular at least 0.3, more specifically at least 0.4 eq base per 1 equivalent of compound (O) are used.

It may be favorable, if, in the synthesis of (T-1), less than 1 eq of base is used in relation to the compound (O). In specific embodiments thereof, NaOH is used as a base, preferably in an amount as given above, in particular in an amount of 0.1 to 0.55 eq in relation to the oxirane of formula (O).

In order to have preferably low reaction times, temperatures of at least 100 °C, more preferably at least 110 °C, in particular at least 120 °C are favorable. It is also an embodiment to reflux the reaction mixture. Preferably, the reaction temperature is not higher than 150°C, in particular not higher than 140°C. Specifically, a reaction temperature of 120°C to 140°C is used.

The amount of 1H-1,2,4-triazole used in step (v) generally is at least 1 eq per mole of oxirane (O). According to one embodiment, the 1H-1,2,4-triazole is used in excess in relation to the oxirane (O). Preferred are more than 1 eq to 2 eq, more preferably more than 1 eq to 1.8 eq, even more preferred more than 1 eq to 1.6 eq. Mostly for economic reason, it can be preferred to use at least 1.1 eq, specifically 1.15 eq, to 1.5 eq of triazole in relation to oxirane (O).

The solvent used in step (v) is preferably selected from dimethylformamide, dimethylacetamide, N-metylpyrrolidone. Most preferred is dimethylformamide.

According to one preferred embodiment, the compounds (T-1) resulting from step (v) are crystallized from a suitable solvent such as, for example toluene, an aliphatic alcohol, acetonitrile, ethyl acetate and/or cyclohexane, in particular toluene and/or an aliphatic alcohol.

Generally, one undesired side product in the synthesis of compounds (T-1) that may occur in undesired amounts is the symmetric triazole (Ts-1) that is formed together with the desired triazole of formula T-1, leading, consequently, to lower yields of the desired product.

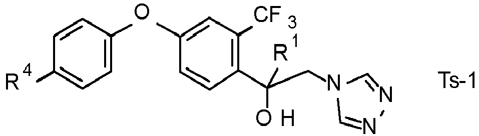

Consequently, according to one preferred embodiment of the invention, the products resulting from step (v) are crystallized from a suitable solvent. This step is called final work up step (v-1). Suitable solvents are, for example, selected from toluene, an aliphatic alcohol, acetonitrile, carbonic acid ester and cyclohexane, or any mixtures thereof, in particular from toluene, an aliphatic alcohol and carbonic acid ester and any mixture thereof.

In particular, the aliphatic alcohol is selected from methanol, ethanol, n-propanol, iso-propanol, n-butanol, isobutanol and any mixture thereof. In particular, the aliphatic alcohol is selected from methanol and ethanol and any mixture thereof.

Examples for suitable carbonic acid esters are n-butyl acetate or ethyl acetate and any mixture thereof.

Generally, for the crystallizing step, the reaction solvent, in particular dimethylformide as described above, is firstly evaporated in large part, preferably under reduced pressure. Preferably, at least 55% of the solvent, more preferably at least 60% of the sovent, more specifically at least 70% of the solvent are removed. Specifically, it may be preferred, if at least 80%, more specifically at least 90% of the solvent, such as DMF, are removed. The solvent can then be recycled to be used again in any previous process step, if necessary after it has been further rectificated before.

Then, water and the respective suitable solvent such as an ether, for example diethylether, diisopropylether, methyl-tert-butylether (MTBE), methylenechloride and /or tolulene, in particular toluene, are added. Also, ethyl acetate and/or n-butyl acetate can be appropriate as solvent. The product I is then preferably obtained by crystallization directly from the concentrated, e.g. toluene-reaction mixture. Also, preferred and suitable according to the invention is the change of solvent to e.g. methanol or ethanol (see above) for the crystallization of the products.

According to one embodiment, seed crystals are added for the crystallization step.

By using the crystallizing step, in particular when carrying out the process steps (v) the formation of the undesired symmetric triazole (Ts-1) as described above can be reduced to equal or less than 10%, more preferably equal or less than 8%, even more preferably equal or less than 5%, even more preferably equal or less than 2%.

Preferably, the ratio of isolated compound (T-1) to the symmetric triazole (Ts-1) is at least 20:1, more preferably at least 30:1, even more preferably 50:1, more specifically 70:1. In particular, the ratio of compound (T-1) to (Ts-1) is at least 30:1.

Also other methods of further reacting the oxiranes (O) to end products (T) can be carried out.

For example, the epoxide ring of compounds (O) may be cleaved by reaction with alcohols R²OH preferably under acidic conditions to result in compounds (VI):

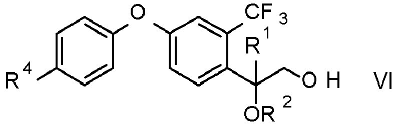

Thereafter, the resulting compounds (VI) are reacted with halogenating agents or sulfonating agents such as PBr₃, PCl₃ mesyl chloride, tosyl chloride or thionyl chloride, to obtain compounds (VII) wherein LG' is a nucleophilically replaceable leaving group such as halogen, alkylsulfonyl, alkylsulfonyloxy and arylsulfonyloxy, preferably chloro, bromo or iodo, particularly preferably bromo or alkylsulfonyl. Then compounds (VII) are reacted with 1H-1,2,4-triazole to obtain compounds (T) as known in the art and/or described above:

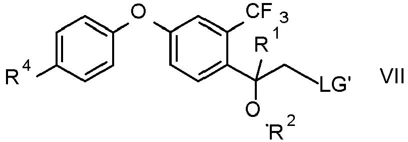

For obtaining compounds of formula T, wherein the alcohol group is derivatized into an ether group to result in compounds of formula T-2, wherein the variables are defined above, the following step can be carried out:
(vi) derivatizing the compound of formula (T-1) as defined in step (v) under basic conditions with R²-LG, wherein LG is a nucleophilically replaceable leaving group; to result in compounds (T-2).

LG represents a nucleophilically replaceable leaving group such as halogen, alkylsulfonyl, alkylsulfonyloxy and arylsulfonyloxy, preferably chloro, bromo or iodo, particularly preferably bromo. Preferably a base is used in step (vi) such as for example, NaH.

Suitable solvents are for example ethers, in particular cyclic ethers. Possible solvents are for example tetrahydrofuran (THF), 2-methyl-tetrahydrofuran (2-Me-THF), diethyl ether, TBME (tert-butyl methyl ether), CPME (cyclopentyl methyl ether), DME (1,2-dimethoxyethane) and 1,4-dioxane. Further solvents that may be suitable are, for example, diisopropyl ether, di-n-butyl ether and/or diglyme. Often, the use of THF or 2-methyl-THF is particularly suitable. Furthermore, it may also be suitable to use combinations of two or more different solvents, such as for example any combination of the solvents listed above or any one of the listed ethers with aliphatic hydrocarbons like n-hexane, heptane or aromatic hydrocarbons like toluene or xylenes.

The skilled person is familiar with the reaction in step (vi) and may vary the reaction conditions analogously to known syntheses.

In one embodiment, a triazole compound of the formula T is obtained by
(vi-a) reacting an oxirane of the formula (O) as defined herein with 1H-1,2,4-triazole and an inorganic base, wherein less than 1 equivalent of said base is used per 1 equivalent of compound (O), resulting in compounds of formula (T).

For obtaining compounds of formula (T-2), wherein the alcohol group is derivatized (resulting in "OR²", see above), the above derivatizing step can be carried out.

### Examples

The following figures and examples further illustrate the present invention and do not restrict the invention in any manner.

### Example 1. Synthesis of 4-nitro-2-(trifluoromethyl)-benzonitrile (K₄Fe(CN)₆·(H2O)₃ / Pd-SPhos 3rd generation)

A solution of 4-nitro-2-(trifluoromethyl)-chlorobenzene (100 mg, 0.443 mmol), KOAc (56.6 mg, 0.576 mmol, 1.3 eq), K₄Fe(CN)₆·(H₂O)₃ (94 mg, 0.222 mmol, 0.5 eq) and 5 mol% Pd-SPhos 3^{rd} generation (17.3 mg, 0.022 mmol, 0.05 equiv) in dioxane (666 µl) and water (666 µl) was prepared in an 8 ml reaction vial in a glove box. The mixture was stirred at 115 °C for 1 hour and analysed via HPLC. The content of 4-nitro-2-(trifluoromethyl)-benzonitrile was 61.8 area %.

### Example 2. Synthesis of 4-nitro-2-(trifluoromethyl)-benzonitrile (Zn(CN)₂ / XPhoS)

A stock solution of Pd₂(dba)₃ (18.3 mg, 20 µmol) and XPhos (19.0 mg, 40 µmol) in toluene (9.0 ml) was stirred at 60 °C for 5-10 minutes. The solution was allowed to cool down to ambient temperature and then a portion of 2.0 ml was added to an 8-ml reaction vial containing NMP (1 mL) and toluene (1 mL). Subsequently 4-nitro-2-(trifluoromethyl)-chlorobenzene (200 mg, 0.89 mmol) was added, and the reaction mixture was stirred at 130 °C in a pre-heated aluminium reaction block. The mixture was stirred at 130 °C for 3 hours and analysed via HPLC. The content of 4-nitro-2-(trifluoromethyl)-benzonitrile was 85.4 area %.

### Example 3. Synthesis of 4-nitro-2-(trifluoromethyl)-benzonitrile (Zn(CN)₂ / XPhoS)

In a glovebox, a stock solution Pd₂(dba)₃ (20.3 mg, 22.2 µmol) and XPhos (21 mg, 44.4 µmol) in toluene (5.0 mL) was prepared in a glovebox. The suspension was stirred for 15 minutes at 115 °C to obtain a brown solution. NaCN (43.5 mg, 0.887 mmol) was weighed in 8 mL screwcap vials. 4-nitro-2-(trifluoromethyl)-chlorobenzene (200 mg, 0.887 mmol), toluene (2.8 ml), the catalyst stock solution (1.0 ml, assumed 8.88 µmol palladium-ligand complex) and NMP (0.2 mL) were added. The vial was heated to 115 °C using a preheated reaction block. A sample was taken after 17 h of reaction time and analyzed as follows: 10 µL of the reaction mixture was dissolved in MeCN (1 mL), filtered (0.45 µm nylon filter) and analyzed by HPLC analysis. The content of 4-nitro-2-(trifluoromethyl)-benzonitrile was 82.9 area %.

### Example 4. Synthesis of 4-nitro-2-(trifluoromethyl)-benzonitrile (CuCN / CuBr₂)

The reaction vials were loaded with a magnetic stirrer, CuCN (1-5 eq.), NMP (1 mL), aryl chloride **1** (500 mg, 2.22 mmol) and CuBr₂ (0.066 eq., where indicated). The resulting reaction mixtures were stirred in closed vessels (under a headspace of N₂) in a heating block of 175 °C and analyzed by HPLC after 40h.

| Entry | CuBr₂ (eq.) | CuCN (eq.) | Product, % |
|---|---|---|---|
| | | | 40h |
| 4.1 | 0 | 5 | 67.8 |
| 4.2 | 0.06 | 5 | 65.6 |

### Example 5. Synthesis of 4-chlorophenoxy-2-trifluoromethyl benzonitrile

A solution of 4-nitro-2-(trifluoromethyl)-benzonitrile (2.00 g, 9.25 mmol), 4-chlorophenol (2.38 g, 18.5 mmol), potassium carbobante (2.56 g, 18.5 mmol) and Cu(OAc)₂ (84 mg, 0.46 mmol) in dry DMF (50 mL) was stirred unter a nitrogen atmosphaere at 97 - 99 °C for 45 min. Ethyl acetate (100 mL) and HCI (1 M aq., 100 mL) were added. The organic layer is washed with brine (3x 100 mL), dried over Na₂SO₄, concentrated under reduced pressure and the resulting crude product was purified via silica chromatography yielding the desired 4-chlorophenoxy-2-trifluoromethyl-benzonitrile in 95% (2.63 g, purity 99.7%).

### Example 6. Synthesis of 1-[4-(4-chlorophenoxy)-2-(trifluoromethyl)phenyl]ethenone

A solution of 3 M MeCI in THF (1.35 mL, 4.03 mmol) in THF (5 mL) was added portion wise to a stirred solution of 4-chlorophenoxy-2-trifluoromethyl-benzonitrile (1.0 g, 3.36 mmol) in THF (10 mL) at ambient temperature. The resulting mixture was heated to 60 °C for 2 h before additional MeMgCI (4.03 mmol) was added as a solution (3 M) in THF and the temperature was elevated to reflux. After a total of 7 h acidic acid (2 mL) and water (20 mL) were added and resulting mixture was stirred for 5 minutes before being added to a mixture of water (30 mL) and ethyl acetate (100 mL). The phases were separated, the organic phase was washed with brine (2x 50 mL) and dried over Na₂SO₄. Volatiles were removed under reduced pressure and the resulting crude product was purified via silica chromatography yielding the desired 1-[4-(4-chlorophenoxy)-2-(trifluoromethyl)phenyl]ethenone in 96% (1.01 g, purity 98.6%).

### Example 7: Synthesis of 2-[4-(4-chlorophenoxy)-2-(trifluoromethyl)phenyl]-2-methyl-oxirane

1-[4-(4-chlorophenoxy)-2-(trifluoromethyl)phenyl]ethenone (0.128 mole) dissolved in 55.4 g dimethylsulfide were provided at 22°C. 25.4 g KOH pellets, 85 wt-% (0.385 mole) were added while stirring heavily. Then, 42.1 g aqueous trimethylsulfonium-methylsulfate (85.6 wt-%, prepared according to Example A 1) were added. This led to an increase of temperature of about 2 to 3°C. Then, it was continued stirring for 8 h at 38°C. A sample of the reaction mixture showed full conversion of the ketone (HPLC). After that, 199 g 20 wt-% NaCl solution was added at 30°C. After separation of the aqueous phase, the dimethylsulfide solution was concentrated by means of distillation of the solvent at a temperature of up to 90°C. 49.7 g of about 82 wt-% ( quant. H PLC) 2-[4-(4-chlorophenoxy)-2-(trifluoromethyl)phenyl]-2-methyl-oxirane were obtained; yield about 97 %.

### Example 8: (Preparation 2-[4-(4-chlorophenoxy)-2-(trifluoromethyl)phenyl]-1-(1,2,4-triazol-1-yl)propan-2-ol

109 g (51.3 wt-% in DMF; 0.1701 mole) 2-[4-(4-chlorophenoxy)-2-(trifluoromethyl)phenyl]-2-methyl-oxirane were diluted with 105.6 g DMF at room temperature. 15.6 g (98 wt-%; 0.221 mole) of 1,2,4-triazole and 3.47 g (0.085 mole) NaOH flakes were added under stirring. The reaction mixture was heated to 125 - 126°C and then stirred for 5 h in total at this temperature. A HPLC-sample showed complete conversion to the desired product (ratio triazol-1-yl/triazol-4-yl about 10:1). About 93% of the DMF was evaporated at 125°C / 300-60 mbar. To the concentrated reaction mixture, 150 g butyl acetate and 92.3 g water were added and the mixture stirred over 10 minutes. Then, the aqueous phase was separated at 80°C.

The organic phase was concentrated at 85°C / 400-130 mbar by 50% (distillate of 117.6 g butyl acetate). The solution was cooled to 60°C and seeded with product and stirred at this temperature over 30 minutes so that the product crystallized slowly. Further cooling to 0°C with a rate of 7.5°K/h followed by suction filtration of the product, washing with 42.8 g n-butyl acetate at 0°C and drying in a drying cabinet at 55°C / 15 mbar led to 52.1 g of product (78.1 % of the theory).

## Claims

1. A process for the preparation of ketone compounds of formula (V) 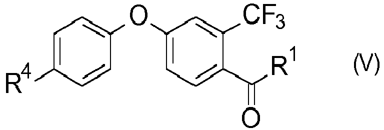 by reacting a nitrile compound of formula (IV) 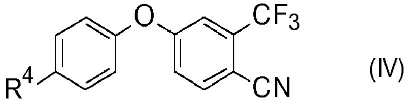 with compounds selected from the group comprising R¹-Mg-Hal, (R¹)Li, (R¹)₂Zn and Al(R¹)₃
wherein the variables are defined as follows:
R¹ is C₁-C₆-alkyl or C₃-C₈-cycloalkyl;
R⁴ is halogen; and
Hal is halogen.

2. The process of claim 1, wherein the compound of formula (IV) is reacted with R¹-Mg-Hal.

3. The process of claim 1, wherein the compound of formula (IV) is reacted with (R¹)Li.

4. The process of claim 1, wherein the compound of formula (IV) is reacted with (R¹)₂Zn.

5. The process of claim 1, wherein the compound of formula (IV) is reacted with Al(R¹)₃.

6. The process of any one of claims 1 to 5, wherein the reaction is carried out in the presence of a catalyst.

7. The process of claim 6, wherein the catalyst is selected from Cu or Ti catalysts.

8. The process of claim 6 or 7, wherein the catalyst is selected from CuBr, CuBr₂, CuCl, CuCl₂, CuI, Cu(OAc)₂, CuCN and Ti(OiPr)₄.

9. The process of any one of claims 6 to 8, wherein the catalyst is used in an amount of from 0.005 to 0.2 mole equivalents per 1 mole of compound (IV).

10. The process of any one of claims 1 to 9, wherein R¹ is selected from CH₃, CH(CH₃)₂ and cyclopropyl.

11. The process of any one of claims 1 to 10, wherein R⁴ is Cl.

12. The process of any one of claims 1 to 11, wherein Hal is Br or Cl, in particular Cl.

13. The process of any one of claims 1 to 12, wherein the compound (IV) is prepared by
(i) reacting the compound of formula (II) 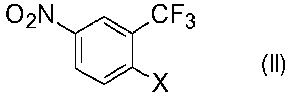
wherein X is halogen
with a cyanide in the form of an inorganic salt, a cyano-metal complex salt or a hydrate thereof, or an organic cyanohydrin,
to give the compound of formula (I) 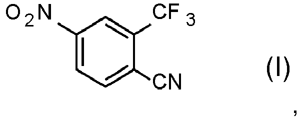
(ii) reacting the compound of formula (I) with a phenol derivative of formula (III) 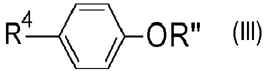 wherein
R" is hydrogen or an alkali or earth alkali metal ion and
R⁴ is halogen;
to give the compound of the formula (IV).

14. A process for the preparation of triazole compounds of formula (T) 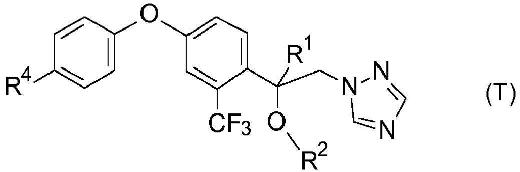 wherein R¹ and R⁴ are defined as in any one of claims 1 to 10 and
R² is hydrogen, C₁-C₆-alkyl, C₂-C₆-alkenyl, C₂-C₆-alkynyl, C₃-C₈-cycloalkyl, C₃-C₈-cycloalkyl-C₁-C₆-alkyl, phenyl, phenyl-C₁-C₄-alkyl, phenyl-C₂-C₄-alkenyl or phenyl-C₂-C₄-alkynyl; wherein the aliphatic moieties of R² are not further substituted or do carry one, two, three or up to the maximum possible number of identical or different groups R^{2a} which independently are selected from:
R^{2a} halogen, OH, CN, nitro, C₁-C₄-alkoxy, C₃-C₈-cycloalkyl, C₃-C₈-halocycloalkyl and C₁-C₄-halogenalkoxy; wherein the cycloalkyl and/or phenyl moieties of R² are not further substituted or do carry one, two, three, four, five or up to the maximum number of identical or different groups R^{12b} which independently are selected from:
R^{2b} halogen, OH, CN, nitro, C₁-C₄-alkyl, C₁-C₄-alkoxy, C₁-C₄-halogenalkyl, C₃-C₈-cycloalkyl, C₃-C₈-halocycloalkyl and C₁-C₄-halogenalkoxy;
comprising the following steps:
(i) preparing compound (IV) according to any one of claims 1 to 13;
(ii) reacting the compound (IV) to oxiranes of formula (O); 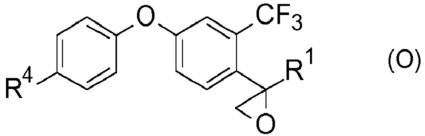
(iii) reacting the oxirane (O) with 1H-1,2,4-triazole in the presence of a base to obtain compounds (I), wherein R² is hydrogen (compounds T-1);
and, for obtaining compounds wherein R² is different from hydrogen:
(iv) derivatizing the compound of formula (T-1) of step (iii) under basic conditions with R²-LG, wherein LG is a nucleophilically replaceable leaving group; to result in compounds (T-2).
